(19)

Europäisches Patentamt
European Patent Office
Office européen des brevets

(11) **EP 4 335 454 A1**

(12) **EUROPEAN PATENT APPLICATION**
published in accordance with Art. 153(4) EPC

(43) Date of publication:
**13.03.2024 Bulletin 2024/11**

(21) Application number: **21888665.3**

(22) Date of filing: **05.11.2021**

(51) International Patent Classification (IPC):
***A61K 38/44*** (2006.01)    ***A61K 47/60*** (2017.01)
***A61P 19/06*** (2006.01)    ***C12N 9/06*** (2006.01)

(52) Cooperative Patent Classification (CPC):
**A61P 13/02; A61K 38/44; A61K 47/00;**
**A61K 47/60; A61P 19/06; C12N 9/0004;**
**C12N 9/0048; C12Y 107/03003**

(86) International application number:
**PCT/CN2021/129071**

(87) International publication number:
**WO 2022/095973 (12.05.2022 Gazette 2022/19)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **05.11.2020 CN 202011224613**

(71) Applicant: **Hangzhou Grand Biologic**
**Pharmaceutical Inc.**
**Hangzhou, Zhejiang 310019 (CN)**

(72) Inventors:
• **HE, Yunfeng**
  **Hangzhou, Zhejiang 310019 (CN)**
• **WANG, Zhiming**
  **Hangzhou, Zhejiang 310019 (CN)**
• **FU, Zhicheng**
  **Hangzhou, Zhejiang 310019 (CN)**
• **WANG, Yu**
  **Hangzhou, Zhejiang 310019 (CN)**
• **WEN, Haiyan**
  **Hangzhou, Zhejiang 310019 (CN)**

• **YAN, Tianwen**
  **Hangzhou, Zhejiang 310019 (CN)**
• **HU, Chunlan**
  **Hangzhou, Zhejiang 310019 (CN)**
• **SU, Guowei**
  **Hangzhou, Zhejiang 310019 (CN)**
• **LIU, Riyong**
  **Hangzhou, Zhejiang 310019 (CN)**
• **DING, Xupeng**
  **Hangzhou, Zhejiang 310019 (CN)**
• **FAN, Kai**
  **Hangzhou, Zhejiang 310019 (CN)**
• **WANG, Hongying**
  **Hangzhou, Zhejiang 310019 (CN)**
• **WANG, Qian**
  **Hangzhou, Zhejiang 310019 (CN)**

(74) Representative: **DehnsGermany Partnerschaft**
**von Patentanwälten**
**Theresienstraße 6-8**
**80333 München (DE)**

(54) **URATE OXIDASE PREPARATION AND USE THEREOF**

(57)    Provided is a urate oxidase preparation. The urate oxidase preparation includes polyethylene glycol-modified urate oxidase as an active ingredient and a buffer reagent as an excipient.

EP 4 335 454 A1

## Description

## TECHNICAL FIELD

The present disclosure relates to the field of biomedicine and, in particular, to urate oxidase preparations and pharmaceutical compositions.

## BACKGROUND

Gout is a disease caused by a long-term disorder of purine metabolism or a decrease in uric acid excretion. Its clinical feature is hyperuricemia, and due to poor solubility of urate, crystal deposits and accumulates subcutaneously and in joints and kidney to form tophus, leading to recurrent acute arthritis, and cause uric acid urinary calculi and interstitial nephritis in kidney. In human body, purine is converted into end-product uric acid through enzyme action. Under normal conditions, the uric acid content in male blood is 149 to 416 mmol/L, the uric acid content in female blood is 89 to 357 mol/L, the body uric acid amount is about 1,200 mg, the production and excretion amount is about 600 mg/day, which is in the equilibrium state. However, hyperuricemia may be caused by excessive intake of uric acid or dysfunction of the excretion mechanism, resulting in the accumulation of uric acid above 70 mg/L in blood. Sodium urate crystallizes out at saturation in blood or synovial fluid, or crystallizes out around joints and soft tissues during long-term hyperuricemia, leading to gouty acute or chronic arthritis and joint deformities. The deposition of urate in renal tubules and interstitium may induce inflammation that leads to chronic urate nephropathy; in patients with severe hyperuricemia (such as patients with malignant tumors such as leukemia and lymphoma), acute renal failure caused by urinary tract obstruction due to massive deposition of uric acid in a short period of time is also called uric acid nephropathy.

The cause of hyperuricemia is related to the mutation and inactivation of the uricase gene during human evolution, so human cannot synthesize active uricase by themselves. One of the current treatments for hyperuricemia is the use of uricase to reduce uric acid levels in patients.

Uricase (E C 1.7.3.3) is widely present in microorganisms (Bacillus fastidious, Candida monocytogenes, Aspergillus flavus), plants (soybeans, chickpeas), animals (pigs, cattle, dogs, baboons) (Suzuki K, Sakasegawa S, Misaki H, Sugiyama M. J Biosci Bioeng. 2004. 98: 153-158), and in the presence of oxygen, it can catalyze uric acid to oxidize allantoin to release carbon dioxide (Retailleau P, Colloc'h, Denis V, Francoise B. Acta Cryst D.2004.60: 453-462.). Active uricase is a tetrameric protein consisting of identical subunits, each with a molecular weight of around 34 kD, consisting of 301 to 304 amino acids. The pH at which the enzyme activity of uricase is highest in each solution is 8.0 (Bayol A et al. Biophys Chem. 1995.54: 229-235.).

Active uricase is a tetrameric protein consisting of identical subunits, each with a molecular weight of around 34 kD, consisting of 301 to 304 amino acids. The pH at which the enzyme activity of uricase is highest in each solution is 8.0 (Bayol A et al. Biophys Chem. 1995.54: 229-235.). Of all the uricases from known sources, the highest activity comes from Aspergillus flavus and reaches 27 IU/mg; the second is derived from Bacillus fastidious, whose activity is maintained at 13 IU/mg (HuangS H, Wu T K. Eur J Biochem. 2004.271: 517-523.). In addition, the activity of uricase from legumes is only 2 to 6 IU/mg; the enzyme activity of uricase derived from mammals, after recombinant expression, can reach 5 IU/mg in pigs, only 1 IU/mg in baboons (Michael H, Susan J. K. 2006. US7056713B1), while human uricase is inactive.

As a human application, the high activity of microbial uricase and low immunogenicity of mammalian uricase make these two sources of uricase become the research focus of recombinant uricase developed and applied at present. However, the homology between Aspergillus flavus-derived uricase and the speculated human-derived uricase is less than 40% (Lee C C, Wu X, Gibbs R A, Cook R G, Muzny D M, CaskeyC T. Science.1988.239: 1288-1291.), the human body is prone to produce anti-uricase antibodies, the efficacy of Aspergillus flavus uricase is rapidly weakened, and severe allergic reactions are triggered. Therefore, the Aspergillus flavus-derived uricase cannot be used for long-term treatment.

As a kind of protein, urate oxidase needs to ensure its enzyme activity, stability, and storage duration when it is prepared into pharmaceutical preparations. Different preparation methods, dosage forms, buffers, and stabilizers will affect the storage duration and activity of urate oxidase, and chemical modification on protein structure will also affect the stability of urate oxidase preparations.

Therefore, it is necessary to develop a urate oxidase preparation that can be stably stored to ensure the enzyme activity of urate oxidase.

## SUMMARY

The present disclosure is based on the discovery and recognition by the inventors of the following facts and problems:

The active urate oxidase is a homotetrameric protein, one-third of the amino acids of which are strongly hydrophobic amino acids, and the tetrameric proteins may easily aggregate to form octamers and larger aggregates. Molecules with a molecular weight above 100 kDa can effectively induce the body to produce an immune response, the molecular weight of the unmodified polymer urate oxidase protein has already reached 140 kDa, and the polymer uricase with greater molecular weight will have higher immunogenicity. The human body is prone to produce antibodies against uricase, thereby rapidly weakening the efficacy thereof and causing severe allergic reactions, and thus the uricase is unsuitable for long-term treatment. It has been proven that through covalent modification of proteins with PEG (polyethylene glycol), the protein immunogenicity can be reduced, the protein solubility can be increased, and the protein half-life can be prolonged.

Duke University and Savient conducted research on chimeric uricase derived from pigs and baboons (Michael H, Susan J.K. 2006. US7056713B1). In this research, ε-amino group of lysine residue of the urease derived from pig-like sources was modified with methoxy-containing polyethylene glycol having a molecular weight of 10 kDa (10 kDa-mPEG-NPC), the modified product is Pegloticase, and thus the goal of treating intractable gout in the human body has been initially achieved without significantly reducing enzyme activity. Applicant found that the above-mentioned research results cannot completely solve the immunogenicity problem caused by the drug. Clinical subjects have experienced the disappearance of the uricase efficacy after multiple injections, which, according to Applicant's speculations, may be related to the excessively great molecular weight of the Pegloticase protein (adopting 10kd PEG, the resulting Pegloticase having a molecular weight of 540 kDa). Further, since pegloticase is not suitable for injection, but suitable for intravenous bolus injection, the subjects' compliance with long-term use is reduced, thereby severely limiting its clinical application. So far, there is no long-acting urate oxidase drug having lower immunogenicity and suitable for subcutaneous injection.

The modification of urate oxidase with polyethylene glycol will change the properties of urate oxidase depending on the modification amount and modification site, and the formula of urate oxidase preparation needs to be changed accordingly to ensure that the enzyme activity, stability, and storage duration of different urate oxidases in the preparation meet the standards.

The present disclosure is intended to solve at least one of the technical problems in the related art to some extent.

In a first aspect of the present disclosure, the present disclosure provides a urate oxidase preparation. According to an embodiment of the present disclosure, the urate oxidase preparation includes: an active ingredient selected from polyethylene glycol-modified urate oxidase; and an excipient selected from a buffer reagent. The buffer reagent includes at least one of phosphate, hydrochloride, or carbonate. The formulation of the preparation according to the embodiment of the present disclosure is simple in formulation, urate oxidase is highly stable under the formulation, and the production of the formulation can save production costs and has a high production efficiency.

In addition, the urate oxidase preparation according to the above embodiment of the present disclosure further has the following additional technical features:

According to an embodiment of the present disclosure, at least 11 of the following amino acid sites in the urate oxidase have a PEG modification: $T^1$, $K^3$, $K^4$, $K^{30}$, $K^{35}$, $K^{76}$, $K^{79}$, $K^{97}$, $K^{112}$, $K^{116}$, $K^{120}$, $K^{152}$, $K^{179}$, $K^{222}$, $K^{231}$, $K^{266}$, $K^{272}$, $K^{285}$, $K^{291}$, and $K^{293}$.

According to an embodiment of the present disclosure, further included is an ultrafiltration and/or purification treatment of a coupling reaction product. In turn, unmodified polyethylene glycol and by-products, such as NHS, can be effectively removed, effectively increasing the purity of the polyethylene glycol-modified urate oxidase obtained.

According to an embodiment of the present disclosure, at least one of the following four amino acid sites has the PEG modification, $K^{30}$, $K^{35}$, $K^{222}$, and $K^{231}$.

According to an embodiment of the present disclosure, the amino acid sites are positioned based on the amino acid sequence set forth as SEQ ID NO: 1.

TYKKNDEVEFVRTGYGKDMIKVLHIQRDGKYHSIKEVATTVQLTLSSKKD

YLHGDNSDVIPTDTIKNTVNVLAKFKGIKSIETFAVTICEHFLSSFKHVIRAQVYVEEVP

WKRFEKNGVKHVHAFIYTPTGTHFCEVEQIRNGPPVIHSGIKDLKVLKTTQSGFEGFI

KDQFTTLPEVKDRCFATQVYCKWRYHQGRDVDFEATWDTVRSIVLQKFAGPYDKGE

YSPSVQKTLYDIQVLTLGQVPEIEDMEISLPNIHYLNIDMSKMGLINKEEVLLPLDNPY

GKITGTVKRKLSSRL (SEQ ID NO:1).

According to an embodiment of the present disclosure, the urate oxidase has an amino acid sequence set forth as any one of SEQ ID NOs: 1 to 7.

MAHYRNDYKKNDEVEFVRTGYGKDMIKVLHIQRDGKYHSIKEVATSVQL
TLSSKKDYLHGDNSDVIPTDTIKNTVNVLAKFKGIKSIETFAVTICEHFLSSFKHVIRAQ
VYVEEVPWKRFEKNGVKHVHAFIYTPTGTHFCEVEQIRNGPPVIHSGIKDLKVLKTTQ
SGFEGFIKDQFTTLPEVKDRCFATQVYCKWRYHQGRDVDFEATWDTVRSIVLQKFAG
PYDKGEYSPSVQKTLYDIQVLTLGQVPEIEDMEISLPNIHYLNIDMSKMGLINKEEVLL
PLDNPYGRITGTVKRKLTSRL (SEQ ID NO:2).

MYKNDEVEFVRTGYGKDMVKVLHIQRDGKYHSIKEVATSVQLTLSSKKD
YVYGDNSDIIPTDTIKNTVHVLAKFKGIKSIETFAMNICEHFLSSFNHVIRAQVYVEEV
PWKRFEKNGVKHVHAFIHNPTGTHFCEVEQMRSGPPVIHSGIKDLKVLKTTQSGFEG
FIKDQFTTLPEVKDRCFATKVYCKWRYHQGRDVDFEATWDTVRDIVLEKFAGPYDK
GEYSPSVQKTLYDIQVHSLSRVPEMEDMEISLPNIHYFNIDMSKMGLINKEEVLLPLDN
PYGKITGTVKRKLSSRL (SEQ ID NO:3).

MAHYHNDYKKNDEVEFVRTGYGKDMVKVLHIQRDGKYHSIKEVATSVQL
TLSSKKDYVYGDNSDIIPTDTIKNTVHVLAKFKGIKSIETFAMNICEHFLSSFNHVIRAQ
VYVEEVPWKRFEKNGVKHVHAFIHNPTGTHFCEVEQMRSGPPVIHSGIKDLKVLKTT
QSGFEGFIKDQFTTLPEVKDRCFATKVYCKWRYHQGRDVDFEATWDTVRDIVLEKFA
GPYDKGEYSPSVQKTLYDIQVHSLSRVPEMEDMEISLPNIHYFNIDMSKMGLINKEEV

LLPLDNPYGRITGTAKRKLASKL (SEQ ID NO:4).

MAHYHNDYQKNDEVEFVRTGYGKDMVKVLHIQRDGKYHSIKEVATSVQL
TLNSRREYLHGDNSDIIPTDTIKNTVQVLAKFKGIKSIETFAMNICEHFLSSFNHVIRVQ
VYVEEVPWKRFEKNGVKHVHAFIHTPTGTHFCEVEQLRSGPPVIHSGIKDLKVLKTT
QSGFEGFLKDQFTTLPEVKDRCFATQVYCKWRYHQGRDVDFEATWEAVRGIVLKKFA
GPYDKGEYSPSVQKTLYDIQVLSLSQLPEIEDMEISLPNIHYFNIDMSKMGLINKEEVL
LPLDNPYGRITGTVKRKLTSRL (SEQ ID NO:5).

MAHYHNDYKKNDEVEFVRTGYGKDMVKVLHIQRDGKYHSIKEVATSVQL TLSSKKDYLHGDNSDIIPTDTIKNTVHALAKFKGIKSIEAFAVNICQHFLSSFNHVIRTQ VYVEEIPWKRLEKNGVKHVHAFIHTPTGTHFCEVEQLRSGPPVIHSGIKDLKVLKTTQ SGFEGFIKDQFTTLPEVKDRCFAAQVYCKWRYHQCRDVDFEATWDTIRDVVLEKFAG PYDKGEYSPSVQKTLYDIQVVSLSQVPEIDDMEISLPNIHYFNIDMSKMGLINKEEVLL PLDNPYGKITGTVKRKLSSRL (SEQ ID NO:6).

MADYHNNYKKNDELEFVRTGYGKDMVKVLHIQRDGKYHSIKEVATSVQL TLSSKKDYLHGDNSDIIPTDTIKNTVHVLAKFKGIKSIEAFGVNICEYFLSSFNHVIRAQ VYVEEIPWKRLEKNGVKHVHAFIHTPTGTHFCEVEQLRSGPPVIHSGIKDLKVLKTTQ SGFEGFIKDQFTTLPEVKDRCFATQVYCKWRYHQCRDVDFEATWGTIRDLVLEKFAG PYDKGEYSPSVQKTLYDIQVLSLSRVPEIEDMEISLPNIHYFNIDMSKMGLINKEEVLLP LDNPYGKITGTVKRKLSSRL (SEQ ID NO:7).

The amino acid sequence set forth as SEQ ID NO: 1 is an amino acid sequence of a chimeric uricase (pig-baboon) derived from pig and baboon; the amino acid sequence set forth as SEQ ID NO: 2 is an amino acid sequence of pig-derived urate oxidase; the amino acid sequence set forth as SEQ ID NO: 3 is an amino acid sequence of a chimeric urate oxidase (canine-baboon) derived from canine and baboon; the amino acid sequence set forth as SEQ ID NO: 4 is an amino acid sequence of canine-derived urate oxidase; the amino acid sequence set forth as SEQ ID NO: 5 is an amino acid sequence of bovine-derived urate oxidase; the amino acid sequence set forth as SEQ ID NO: 6 is an amino acid sequence of monkey-derived urate oxidase; and the amino acid sequence set forth as SEQ ID NO: 7 is an amino acid sequence of baboon-derived urate oxidase.

It should be noted that lysine in the present disclosure is positioned based on the amino acid sequence set forth as SEQ ID NO: 1. For example, $K^4$ refers to the lysine located at position 4 based on the amino acid sequence set forth as SEQ ID NO: 1. The uricase has the amino acid sequence set forth as any one of SEQ ID NOs: 1 to 7, the polypeptide having at least 70%, at least 75%, at least 80%, at least 85%, at least 90%, at least 95%, or at least 99% identity with any one of SEQ ID NOs: 1 to 7, and the polypeptide having the amino acid sequence set forth as any one of SEQ ID NOs: 1 to 7 in which one or more amino acids are substituted, deleted and/or added have homology in their structures. Those skilled in the art, through sequence mapping, can determine respective positions corresponding to $T^1$, $K^3$, $K^4$, $K^{30}$, $K^{35}$, $K^{76}$, $K^{79}$, $K^{97}$, $K^{112}$, $K^{116}$, $K^{120}$, $K^{152}$, $K^{179}$, $K^{222}$, $K^{231}$, $K^{266}$, $K^{272}$, $K^{285}$, $K^{291}$, and $K^{293}$ on any one of SEQ ID NOs: 2 to 7, or the polypeptide having at least 70%, at least 75%, at least 80%, at least 85%, at least 90%, at least 95%, or at least 99% identity with any one of SEQ ID NOs: 1 to 7, or the polypeptide having the amino acid sequence set forth as any one of SEQ ID NOs: 1 to 7 in which one or more amino acids are substituted, deleted and/or added, and then allow the PEG modification to occur at the corresponding positions on the above-mentioned polypeptide sequence, thereby achieving the advantages of the polyethylene glycol-modified urate oxidase of the present disclosure, such as low immunogenicity, high stability in vivo, and applicability for intramuscular injection.

For example, according to the embodiments of the present disclosure, sites of the sequence set forth as SEQ ID NO: 2 corresponding to sites $T^1$, $K^3$, $K^4$, $K^{30}$, $K^{35}$, $K^{76}$, $K^{79}$, $K^{97}$, $K^{112}$, $K^{116}$, $K^{120}$, $K^{152}$, $K^{179}$, $K^{222}$, $K^{231}$, $K^{266}$, $K^{272}$, $K^{285}$, $K^{291}$, and $K^{293}$ of the sequence set forth as SEQ ID NO: 1 include $M^1$, $K^9$, $K^{10}$, $K^{36}$, $K^{41}$, $K^{82}$, $K^{85}$, $K^{103}$, $K^{118}$, $K^{122}$, $K^{126}$, $K^{158}$, $K^{185}$, $K^{228}$, $K^{237}$, $K^{272}$, $K^{278}$, $K^{297}$, and $K^{299}$; sites of the sequence set forth as SEQ ID NO: 3 corresponding to the above-mentioned respective sites of the sequence set forth as SEQ ID NO: 1 include $M^1$, $K^3$, $K^{29}$, $K^{34}$, $K^{75}$, $K^{78}$, $K^{111}$, $K^{115}$, $K^{119}$, $K^{151}$, $K^{118}$, $K^{221}$, $K^{230}$, $K^{265}$, $K^{271}$, $K^{284}$, $K^{290}$, and $K^{292}$; sites of the sequence set forth as SEQ ID NO: 4 corresponding to the above-mentioned respective sites of the sequence set forth as SEQ ID NO: 1 include $M^1$, $K^9$, $K^{10}$, $K^{36}$, $K^{41}$, $K^{82}$, $K^{85}$, $K^{118}$, $K^{122}$, $K^{126}$, $K^{158}$, $K^{185}$, $K^{228}$, $K^{237}$, $K^{272}$, $K^{278}$, $K^{297}$, and $K^{299}$; sites of the sequence set forth as SEQ ID NO: 5 corresponding to the above-mentioned respective sites of the sequence set forth as SEQ ID NO: 1 include $M^1$, $K^{10}$, $K^{36}$, $K^{41}$, $K^{82}$, $K^{85}$, $K^{118}$, $K^{122}$, $K^{126}$, $K^{158}$, $K^{185}$, $K^{228}$, $K^{237}$, $K^{272}$, $K^{278}$, $K^{297}$, and $K^{299}$; sites of the

sequence set forth as SEQ ID NO: 6 corresponding to the above-mentioned respective sites of the sequence set forth as SEQ ID NO: 1 include $M^1$, $K^9$, $K^{10}$, $K^{36}$, $K^{41}$, $K^{82}$, $K^{85}$, $K^{118}$, $K^{122}$, $K^{126}$, $K^{158}$, $K^{185}$, $K^{228}$, $K^{237}$, $K^{272}$, $K^{278}$, $K^{291}$, $K^{297}$, and $K^{299}$; sites of the sequence set forth as SEQ ID NO: 7 corresponding to the above-mentioned respective sites of the sequence set forth as SEQ ID NO: 1 include $M^1$, $K^9$, $K^{10}$, $K^{36}$, $K^{41}$, $K^{82}$, $K^{85}$, $K^{118}$, $K^{122}$, $K^{126}$, $K^{158}$, $K^{158}$, $K^{228}$, $K^{237}$, $K^{272}$, $K^{278}$, $K^{291}$, $K^{297}$, and $K^{299}$. The inventors found through experiments that, after at least 11 sites of the respective sites of the amino acid sequence set forth as any one of SEQ ID NOs: 2 to 7 are modified with PEG, the obtained PEG-modified urate oxidase has low immunogenicity, high stability in vivo, and applicability for intramuscular injection.

According to an embodiment of the present disclosure, peak areas of at least 11 predetermined peptide fragments in a peptide map of the polyethylene glycol-modified urate oxidase are reduced by a relative proportion of 75% or more, preferably 80% or more, more preferably 90% or more, compared with those in a peptide map of urate oxidase unmodified with polyethylene glycol. The polyethylene glycol-modified urate oxidase according to the embodiment of the present disclosure has the advantages of low immunogenicity, high stability in vivo, and applicability for intramuscular injection.

According to an embodiment of the present disclosure, the molecular weight of the polyethylene glycol used for PEG modification is not more than 6 KD. The inventors have found that the modification with polyethylene glycol having a molecular weight of not more than 6 KD further enhances the long-lasting efficacy in vivo of the urate oxidase obtained, without generating severe anti-PEG antibodies due to excessively large molecular weight, i.e., with further reduced immunogenicity.

According to an embodiment of the present disclosure, the polyethylene glycol has a monomethoxy group or a hydroxyl group.

According to an embodiment of the present disclosure, the polyethylene glycol is of a linear or branched structure.

According to an embodiment of the present disclosure, the polyethylene glycol is coupled to the urate oxidase via an amide bond.

According to an embodiment of the present disclosure, the polyethylene glycol is a modified polyethylene glycol, and a modification group of the modified polyethylene glycol comprising at least one selected from the group consisting of N-hydroxysuccinimide, N-hydroxysuccinimidyl carbonate, N-hydroxysuccinimidyl acetate, N-hydroxysuccinimidyl propionate, N-hydroxysuccinimidyl butyrate, N-hydroxysuccinimidyl succinate, and bis(p-nitrophenyl) carbonate.

According to an embodiment of the present disclosure, the modification group of the modified polyethylene glycol is N-hydroxysuccinimidyl propionate.

According to the method of the embodiment of the present disclosure, the immunogenicity of urate oxidase can be effectively reduced, and the resulting urate oxidase has higher in vivo safety and a better long-lasting effect.

It can be understood that the additional technical features of the above-mentioned method for preparing polyethylene glycol-modified urate oxidase and the technical effects possessed by the additional technical features are applicable to the additional technical features of the method for reducing the immunogenicity of urate oxidase according to the embodiments of the present disclosure, and the additional technical features of the method for reducing the immunogenicity of urate oxidase according to the embodiments of the present disclosure will not be described in detail herein.

According to an embodiment of the present disclosure, the buffer reagent includes at least one of disodium hydrogen phosphate, sodium dihydrogen phosphate monohydrate, or sodium chloride. According to the urate oxidase preparation of the embodiments of the present disclosure, using disodium hydrogen phosphate, sodium dihydrogen phosphate monohydrate, and sodium chloride as the excipient can ensure the enzyme-specific activity of urate oxidase, and the specific activity, protein degradation, and aggregation degree of the enzyme stored at low temperature, normal temperature, and high temperature for 30 days meet expectations. Furthermore, the method of the present disclosure is simple in the formulation and has high stability compared with the conventional urate oxidase preparation requiring the addition of glycine, sucrose, and the like.

According to an embodiment of the present disclosure, the buffer reagent refers to a buffer that resists a change in pH through the action of its acid-base conjugate component. The buffer reagent may be present in the liquid or solid preparations of the present disclosure and adjusts the pH of the preparation to 7 to 9. The buffer reagent controlling the pH in the range of 7 to 9 includes acetate, succinate, gluconate, histidine, citrate, phosphate, maleate, dimethylarsinate, 2-(N-morpholino)ethanesulfonic acid (MES), bis-(2-hydroxyethyl) amino (hydroxymethyl) methane (Bis-Tris), N-(carbamoylmethyl) iminodiacetic acid (ADA), glycylglycine, and other organic acid buffers, individually or in combination.

According to an embodiment of the present disclosure, the pH of the urate oxidase preparation is in a range of 7 to 9, preferably 7.4 to 8.2. Through repeated experiments and analysis, the inventors found that the polyethylene glycol-modified urate oxidase preparation has high stability under the above-mentioned pH conditions, the urate oxidase is not prone to aggregate and degrade, and the enzyme-specific activity is high.

According to an embodiment of the present disclosure, the pH of the uricase preparation is 7.4, 7.5, 7.6, 7.7, 7.8, 7.9, 8.0, 8.1, or 8.2.

According to an embodiment of the present disclosure, a mass ratio of the polyethylene glycol-modified urate oxidase to the buffer reagent is (5 to 6): (6 to 37), preferably 6:10. According to the urate oxidase preparation of the embodiments of the present disclosure, formulating the preparation in the above-mentioned ratio can ensure that the pH of the preparation is 7.4 to 8.2 to ensure the stability of the urate oxidase.

According to an embodiment of the present disclosure, the mass ratio of the polyethylene glycol-modified urate oxidase to the buffer reagent is 6:10, 6:11, 5:10, 5:11, 5:9, or 6:9.

According to an embodiment of the present disclosure, a mass ratio of the polyethylene glycol-modified urate oxidase to the phosphate is (5 to 6): (1 to 7).

According to an embodiment of the present disclosure, a mass ratio of the polyethylene glycol-modified urate oxidase to the sodium chloride is (5 to 6): (5 to 30).

According to an embodiment of the present disclosure, a mass ratio of the phosphate to the sodium chloride is (1 to 7): (5 to 30), wherein the phosphate is disodium hydrogen phosphate and/or sodium dihydrogen phosphate.

According to an embodiment of the present disclosure, in the urate oxidase preparation provided in the present disclosure, it is unnecessary to add stabilizers such as glycerol, glucose, mannitol, and Tween-80, and only the buffer is used to ensure the stability of the urate oxidase provided in the present disclosure, so as to obtain the urate oxidase preparation with high stability. The addition of mannitol and/or glycerol to the urate oxidase according to the embodiments of the present disclosure increases the particle size of the urate oxidase, while the addition of Tween-80 has no significant effect on the stability of the urate oxidase according to the embodiments of the present disclosure. According to an embodiment of the present disclosure, a dosage form of the urate oxidase preparation includes at least one of liquid, semi-solid, or solid. The urate oxidase preparation according to the embodiment of the present disclosure may be a liquid preparation or a lyophilized dosage form, which is dissolved into a liquid at the time of use. Further, the urate oxidase preparation is an injection preparation, which may be injected into the body of a patient by intravenous injection, intramuscular injection or the like.

According to an embodiment of the present disclosure, the preparation is in a single-dose form, each dose containing 6 mg of the urate oxidase. The urate oxidase preparation in a single-dose form according to the embodiment of the present disclosure is administered in a simple manner, does not require repeated administrations every day, and is convenient for a patient to use, thereby achieving maximum efficacy and easy storage.

In a second aspect, the present disclosure provides the use of the urate oxidase preparation provided in the first aspect of the present disclosure in the manufacture of a medicament for the treatment or prevention of hyperuricemia and hyperuricemia-related diseases. According to an embodiment of the present disclosure, the hyperuric acid-related diseases include chronic hyperuricemia, gout, kidney disease, hyperuricemic arthritis, renal calculi, tophus, hypertension, diabetes, hypertriglyceridemia, metabolic syndrome, coronary heart disease, atherosclerosis, and hyperuricemia caused by cancer chemotherapy.

In a third aspect of the present disclosure, a pharmaceutical composition is provided. According to an embodiment of the present disclosure, the pharmaceutical composition includes the urate oxidase preparation provided in the first aspect of the present disclosure.

According to an embodiment of the present disclosure, the pharmaceutical composition further includes at least one of the following additional technical features:

According to an embodiment of the present disclosure, the above pharmaceutical composition further includes an additional drug for treating or preventing hyperuricemia and hyperuricemia-related diseases.

According to an embodiment of the present disclosure, when the polyethylene glycol-modified urate oxidase or pharmaceutical composition of the present disclosure is administered in combination therapy with other drugs, they may be administered to a subject sequentially or simultaneously. Alternatively, a pharmaceutical composition of the present disclosure may include a combination of the polyethylene glycol-modified urate oxidase of the present disclosure, a pharmaceutically acceptable carrier or a pharmaceutically acceptable excipient, and other therapeutic or prophylactic agents known in the art.

According to an embodiment of the present disclosure, the pharmaceutical composition has the advantages of low immunogenicity, high in vivo stability, applicability for intramuscular injection, and can be used for the treatment or prevention of hyperuric acid-related diseases.

According to an embodiment of the present disclosure, the pharmaceutical composition further includes a pharmaceutically acceptable adjuvant. The adjuvant includes any solvent, solid excipient, diluent, binder, disintegrant, or other liquid excipients, dispersing agent, flavoring or suspending agent, surfactant, isotonic agent, thickening agent, emulsifying agent, preservative, solid binder, glidant or lubricant, etc., as suited to the particular dosage form desired.

Additional aspects and advantages of the present disclosure will be set forth in part in the description which follows and, and in part will become apparent from the following description, or may be learned by practice of the present disclosure.

**BRIEF DESCRIPTION OF DRAWINGS**

The foregoing and/or additional aspects and advantages of the present disclosure will become apparent and readily appreciated from the following description of the embodiments, taken in conjunction with the accompanying drawings of which:

FIG. 1 is a spectrum of SEC-HPLC-UV detection of a PHC physical and chemical reference substance according to an embodiment of the present disclosure;
FIG. 2 is a spectrum of SEC-HPLC-RI detection of a PHC physical and chemical reference substance according to an embodiment of the present disclosure;
FIG. 3 is a spectrum of SEC-HPLC-RI detection of a PEG reference substance according to an embodiment of the present disclosure;
FIG. 4 is a spectrum of SEC-HPLC-UV detection of a PU5 modification product according to an embodiment of the present disclosure;
FIG. 5 is a spectrum of SEC-HPLC-RI detection of a PU5 modification product according to an embodiment of the present disclosure;
FIG. 6 is a comparison diagram of PHC and PU5 using Lys-c and trypsin double digestion according to an embodiment of the present disclosure;
FIG. 7 is a diagram of Lys-C digestion of PU5 according to an embodiment of the present disclosure;
FIG. 8 is an SEC (size exclusion chromatography) plot of Formulation 5 at day 0 according to an embodiment of the present disclosure;
FIG. 9 is an SEC plot of Formulation 5 at 37°C on day 30 according to an embodiment of the present disclosure;
FIG. 10 is an SEC plot of Formulation 6 at 37°C on day 30 according to an embodiment of the present disclosure;
FIG. 11 is an SEC plot of Formulation 7 at 37°C on day 30 according to an embodiment of the present disclosure.
FIG. 12 is a graph showing serum uric acid levels after intramuscular administration of different doses to model rats according to an embodiment of the present disclosure;
FIG. 13 is a diagram illustrating scores of kidney injury, necrosis, and inflammation according to an embodiment of the present disclosure;
FIG. 14 is a graph illustrating mean serum drug concentration-time curve of various groups after a single intravenous injection of the same dose (1.0 mg/kg) of Pegloticase and a pegylated uricase injection in SD rats according to an embodiment of the present disclosure;
FIG. 15 illustrates mean serum drug concentration-time curves of various groups after a single intramuscular injection of Pegloticase and pegylated uricase injections of different doses in SD rats according to an embodiment of the present disclosure;
FIG. 16 illustrates mean serum drug concentration-time curves of various groups after a single intramuscular injection of pegylated uricase injections of different doses in SD rats according to an embodiment of the present disclosure;
FIG. 17 illustrates mean serum uric acid value-time curves of various groups after a single intramuscular/intravenous injection of Pegloticase and pegylated uricase injections of different doses in SD rats according to an embodiment of the present disclosure;
FIG. 18 illustrates mean serum drug concentration-time curves of male and female SD rats after the first intravenous injection (Day 1) of the same dose (1.0 mg/kg) of Pegloticase and a pegylated uricase injection according to an embodiment of the present disclosure;
FIG. 19 illustrates mean serum drug concentration-time curves of male and female SD rats after the last intravenous injection (Day 22) of the same dose (1.0 mg/kg) of Pegloticase and a pegylated uricase injection according to an embodiment of the present disclosure;
FIG. 20 illustrates mean serum drug concentration-time curves of male and female SD rats after the first intramuscular injection (Day 1) of the same dose (1.0 mg/kg) of Pegloticase and a pegylated uricase injection according to an embodiment of the present disclosure;
FIG. 21 illustrates mean serum drug concentration-time curves of male and female SD rats after the last intramuscular injection (Day 22) of the same dose (1.0 mg/kg) of Pegloticase and a pegylated uricase injection according to an embodiment of the present disclosure;
FIG. 22 illustrates mean serum uric acid value-time curves after multiple intravenous injections of Pegloticase and a pegylated uricase injection in SD rats according to an embodiment of the present disclosure; and
FIG. 23 illustrates mean serum uric acid value-time curves after multiple intramuscular injections of Pegloticase and a pegylated uricase injection in SD rats according to an embodiment of the present disclosure.

**DESCRIPTION OF EMBODIMENTS**

The embodiments of the present disclosure are described in detail below, and examples of the embodiments are illustrated in the accompanying drawings, throughout which same or similar reference numerals represent same or similar components or components with same or similar functions. The embodiments described below with reference to the accompanying drawings are illustrative and are intended to explain the present disclosure, but should not be understood as a limitation of the present disclosure.

An object of the present disclosure is to provide a novel polyethylene glycol-modified urate oxidase preparation.

Another object of the present disclosure is to provide the use of the polyethylene glycol-modified urate oxidase preparation described above, which can achieve a long-lasting efficacy in vivo and a significant reduction in the serum uric acid level, and can be used for the treatment of hyperuricemia and gout.

In one aspect of the present disclosure, provided is a polyethylene glycol-modified urate oxidase preparation.

Urate oxidase is not particularly limited, and can be urate oxidase and urate oxidase analogues derived from any source. Representative examples include, but are not limited to, mammalian sources, microorganisms, plants, etc.

The urate oxidase derived from different species according to the present disclosure can be obtained through various manners, including but not limited to natural extraction, chemical synthesis, genetic engineering recombinant expression, etc.

In another preferred embodiment, the urate oxidase is prepared and obtained in a manner that E. coli or yeast is used as a host to construct a recombinant expression strain, and E. coli is more preferably used as host bacteria for recombinant expression.

In another preferred embodiment, the polyethylene glycol-modified urate oxidase is used as an active ingredient, and sodium dihydrogen phosphate, disodium hydrogen phosphate, and sodium chloride are added to prepare a preparation with maximized enzyme activity and improved storage stability.

In another preferred embodiment, the polyethylene glycol-modified urate oxidase preparation has high storage stability without adding other stabilizers, such as mannitol, glycerol, and Tween-80, and thus the urate oxidase preparation has a simple formulation, simple preparation process, and low cost.

In one aspect of the present disclosure, provided is the use of the polyethylene glycol-modified urate oxidase preparation as described above.

The polyethylene glycol-modified urate oxidase preparation is more suitable as a drug and composition thereof for treating chronic hyperuricemia or gout. The main symptoms of hyperuricemia and gout include, but are not limited to, uric acid nephropathy and gouty arthritis.

The administration route of the polyethylene glycol-modified urate oxidase includes, but is not limited to, intravenous injection, subcutaneous injection, intramuscular injection, intraperitoneal injection, etc., preferably intravenous injection, intramuscular injection, more preferably intramuscular injection.

The polyethylene glycol-modified urate oxidase has lower immunogenicity in vivo.

The polyethylene glycol-modified urate oxidase having low immunogenicity means that after intramuscular injection of the polyethylene glycol-modified urate oxidase in the human or animal body, the body does not produce antibodies against polyethylene glycol molecules or produces low titer antibodies against polyethylene glycol molecules, and does not produce antibodies against urate oxidase either.

The polyethylene glycol-modified urate oxidase has a longer half-life in vivo and the efficacy in reducing the uric acid level in the blood after intramuscular injection.

Furthermore, the terms "first" and "second" are used for descriptive purposes only and are not to be construed as indicating or implying relative importance or implicitly indicating the number of technical features indicated. Thus, a feature defined as "first" or "second" may explicitly or implicitly include at least one such feature. In the description of the present disclosure, "plurality" means at least two, e.g., two, three, etc., unless specifically limited otherwise.

The present disclosure will now be described with reference to specific examples. It should be noted that these examples are intended to be illustrative only and not limiting in any way.

**Example 1 Preparation of recombinant urate oxidase**

1.1 Construction of genes and expression plasmids for uricase expression

According to the usage preference data of E. coli codon, in combination with factors such as codon preference and GC content, cDNA sequence of the uricase protein (referred to as PHC) (SEQ ID NO:1) was designed, and the whole gene was synthesized and named as pUC-57-PHC plasmid. Nde I and BamH I were used as the target gene insertion sites, and the pET-30a plasmid was used as the expression vector (pET-30a-PHC).

1.2 Transformation of expression plasmids into bacterial host cells

The expression vector pET-30a-PHC was introduced into E. coli BL21 (DE3) by CaCl2 method, high expression clones were screened through resistance screening with Kanamycin, and the original seed bank strain (E3B) was preserved. These steps were performed in accordance with the commonly used methods in the field of molecular biology.

1.3 Preparation of recombinant urate oxidase

The transformed and engineered strains were fermented and expressed in a fermentor, under the control conditions: first culturing at 30°C and pH 7.2 to an $OD_{600}=30$ or higher, adding IPTG to 0.5 mmol/L, and continuing to induce for 3 hours or more to allow urate oxidase to accumulate. The cells were collected by centrifugation, and then preserved below -15°C.

The frozen bacteria were taken and suspended in a buffer of 25 mmol/L Tris and 5 mmol/L EDTA, at a suspension ratio of 1:10 (W/V). After breaking the bacterial cells with high pressure, the urate oxidase precipitate was collected by centrifugation, the precipitate was washed once with 50 mmol/L $NaHCO_3$, and the enriched uricase precipitate was suspended in a $Na_2HCO_3$ buffer (100 mmol/L, pH 9.7 to 10.3) at a suspension ratio of 1:50 (W/V), following by stirring overnight at room temperature to dissolve, and then centrifuging to collect the supernatant.

Urate oxidase was further purified through several chromatographic steps. The purity detected by SDS-PAGE was 95% or greater, and the purity detected by Superdex 200 column was greater than 95%, with no aggregate form. The protein concentration was determined by Lowry method, and the activity of the urate oxidase was measured by spectrophotometry, where 1 unit (U) of enzyme activity was defined as the amount of enzyme required to convert 1 $\mu$mol of uric acid per minute under the optimal reaction temperature of 37°C and the optimal buffer condition at pH 9.0.

**Example 2 Preparation of Pegylated Urate Oxidase**

Monomethoxy PEG derivatives with different molecular weights (500 Da to 20,000 Da), such as N-succinimidyl propionate PEG (5K-PEG-SPA) with a molecular weight of 5 K, are dissolved with a 1 to 5 mmol/L acid solution to form a 100 to 300 mmol/L PEG solution, which, after the dissolving, was added to a carbonate buffer solution containing the urate oxidase dissolved therein and having a carbonate concentration of 0.1 to 0.3 mol/L, pH 10.0, according to molar ratio of 1:45 to 1:150 (urate oxidase: 5K-PEG-SPA), thereby allowing a coupling reaction between PEG and urate oxidase. A concentration of the urate oxidase in the coupling reaction was 10 mg/ml. The coupling reaction required stirring at 5 to 30°C for 60 minutes or more, until the PEG coupling degree no longer changed with time. After the reaction was finished, the PEG not participating in the modification and by-products were removed from the reaction by ultrafiltration and/or chromatography. A suitable molecular sieve chromatography medium was used to separate and remove modified by-products. Finally, the 5K modified pegylated urate oxidase (referred to as PU5) was obtained through sterile filtration.

**Example 3: Characteristic Analysis of Pegylated Urate Oxidase**

3.1 Detection of average modification degree and enzyme activity

The protein concentration was determined by using Lowry method, and the activity of the polyethylene glycol-modified urate oxidase was determined by using spectrophotometer. The maximum ultraviolet absorption wavelength of uric acid, i.e., the substrate of uricase, was 293nm, and the maximum ultraviolet absorption wavelength of the product allantoin was 224nm. Within a certain concentration range, the absorption value of uric acid at 293nm was in direct proportion to its concentration. The quantitative determination of uric acid can be carried out by spectrophotometer. The specific process was as follows: the UV-Vis spectrophotometer was turned on, the wavelength was adjusted to 293nm, and the water bath circulation system of the instrument was turned on to keep the temperature at 37°C. Sodium tetraborate buffer was used as a blank control, and zero point was calibrated; 2.95ml of substrate reaction solution (0.1 mol/L sodium tetraborate, 100 $\mu$mol/L uric acid, pH 9.5, preheated to 37°C) was added in a quartz cuvette, then 50 $\mu$l of the test sample was added and mixed quickly to measure the absorption value at 293nm. The change of the absorbance at 293nm was continuously measured; a degradation concentration of uric acid was calculated according to $C=A/\varepsilon L$ (where A is an absorbance of a specific concentration of uric acid at 293 nm, $\varepsilon$ is a molar extinction coefficient of uric acid, L is an optical path of the cuvette, and C is a molar concentration of uric acid). The enzyme activity was calculated. The enzyme activity was defined as that, at the optimum reaction temperature of 37°C and the optimum reaction pH 9.5, the amount of enzyme required to convert 1 $\mu$mol of uric acid into allantoin per minute is defined as one activity unit (U).

SEC-HPLC connected in series with UV/RI (a combination of ultraviolet and refractive index detector) was used to detect the average modification degree of polyethylene glycol-modified urate oxidase. Based on the fact that protein has a maximum absorption peak at ultraviolet 280nm, PEG has no absorption at this wavelength, and the absorption value of the protein and the absorption value of PEG within a certain range by the differential refractive index detector are proportional to the respective concentrations, the content of PEG portion and the content of protein portion in the pegylated urate oxidase can be obtained using an external standard method with PEG reference substance and PHC physical and chemical reference substance, and thus the number of PEG molecules on each urate oxidase monomer, i.e., the average modification degree, can be obtained by the following calculation method.

Average modification degree of PEG-modified urate oxidase = (relative molecular weight of urate oxidase subunit × amount of PEG in sample)/(relative molecular weight of PEG × amount of protein in sample).

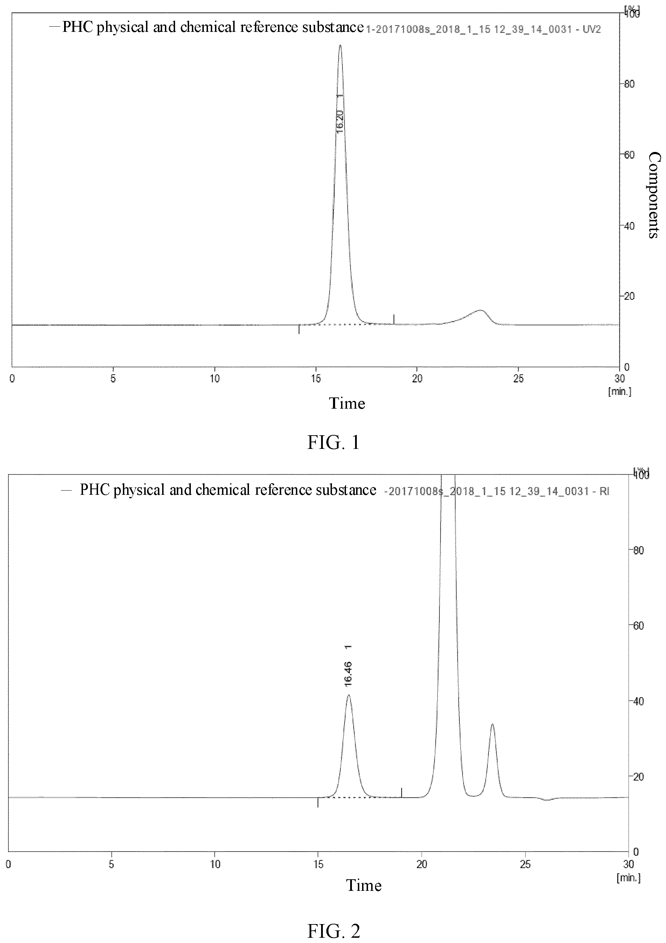

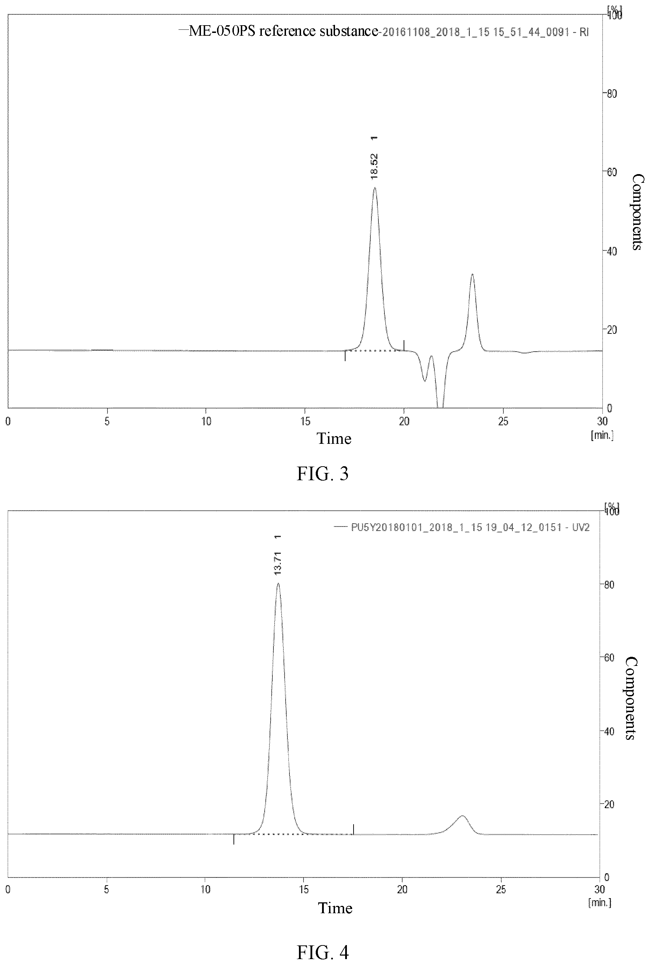

The SEC-HPLC-UV/RI detection spectrums of PHC physical and chemical reference substance, PEG reference substance, and PU5 modified product are illustrated in FIG. 1 to FIG. 5.

Under different feed ratios in Example 2, the enzyme activity and average modification degree of the obtained polyethylene glycol-modified urate oxidase are shown in Table 1.

Table 1 Enzyme activity and average modification degree at different feed ratios at 5K-PEG

| Protein: 5K-PEG feed molar ratio | Enzyme activity | Enzyme activity retention | Average modification degree |
|---|---|---|---|
| Unmodified urate oxidase | 11.4 U/ mg | 100% | 0 |
| 1:48 | 10.71 U/mg | 94% | 10.3 |
| 1:56 | 11.17 U/mg | 103.4% | 11.4 |
| 1:68 | 12.2 U/mg | 107.1% | 11.9 |
| 1:82 | 12.02 U/mg | 105.4% | 12.3 |
| 1:94 | 11.75 U/mg | 103.1% | 12.1 |
| 1:110 | 10.83 U/mg | 95% | 11.5 |
| 1:150 | 10.03 U/mg | 88% | 10.1 |
| [0115] Notes: the average modification degree represents the number of PEG molecules bound per urate oxidase monomer. | | | |

It can be seen from Table 1 that the polyethylene glycol-modified urate oxidase of the present disclosure, under a feed ratio of protein : 5K-PEG being in the range of 1:56 to 1:110, has an average modification degree of more than 11, and the enzyme activity was higher than that of unmodified urate oxidase, the retention rate of enzyme activity was high, the enzyme activity was not decreased, but increased, and the enzyme activity was relatively stable, which is completely different from the marketed drug Krystexx (pegloticase). According to the content disclosed in the patent of Savient (CN1264575C, Figure 2A to Figure 3B) and the common knowledge of those skilled in the art, the enzyme activity decreases significantly with the increasing modification degree of 5kD PEG. However, unexpectedly, with the average modification degree of more than 11, the polyethylene glycol-modified uricase obtained in the present disclosure does not have its enzyme activity changed significantly compared with the unmodified state. Therefore, the polyethylene glycol-modified urate oxidase of the present disclosure has a higher average modification degree of polyethylene glycol compared with the marketed drug, and also achieves unexpected technical effects in terms of enzyme activity retention, which may be attributed to the differences in PEG modification degree or modification sites of polyethylene glycol-modified urate oxidase, according to Applicant's speculation.

3.2 Detection of polyethylene glycol modification sites

In the following procedure, the inventors performed modification site detection on the urate oxidase obtained in Example 2.

The PEG modification sites of polyethylene glycol-modified urate oxidase can be detected by performing enzyme digestion of the non-pegylated and pegylated urate oxidases with one or more enzymes and then performing chromatographic detection to obtain a chromatogram, i.e., peptide map for determination. The non-pegylated and pegylated urate oxidases can be digested through single-enzyme digestion (Lys-C or Trypsin) and/or double-enzyme digestion (Lys-C and Trypsin combined). The digested enzyme fragments were separated by reversed-phase column, and the modified sites of polyethylene glycol-modified urate oxidase were determined by the internal reference peptide fragment correction and comparison of the disappearance or reduction proportion of the peptide fragments.

Modification site analysis principle of trypsin and Lys-C double enzyme digestion quality peptide map: Lys-C can specifically digest the C-terminal of lysine (K); trypsin takes the basic amino acids, i.e., arginine (R) and lysine (K), as restriction enzyme cutting sites, and specifically digests the C-terminal peptide bond. Comparing the changes of the corresponding peptide fragments before and after digestion in PHC and PU5, and with reference to the internal standard peptide fragments, the relative proportion of the reduction or disappearance of the PEG-modified peptide fragments can be analyzed and determined. Through the relative proportion of the reduction or disappearance of the peptide fragment, it can be determined whether the lysine site on the peptide fragment is modified by PEG and the modified proportion. It should be pointed out that PEG modification is a non-uniform modification, and a high modification proportion of a site can be regarded as that this site is modified.

Details as follows:

(1) Sample processing: urate oxidase and pegylated urate oxidase were taken and respectively diluted to 1 mg/ml with enzyme digestion buffer (25mmol/L Tris-HCl, 20% acetonitrile, pH 9.0), and 100 μl of each dilute solution was taken, added with 2 μl of Lys-C, and digested at 37°C for 4 hours. Subsequently, the solution was transferred to a pancreatin reaction tube (in a ratio of 1: 100), digestion was continued at 37°C for 2 hours, 4 μl of TCEP reducing solution was added, the reaction continued for 30 minutes, and then 10 μl of 1 mol/L hydrochloric acid solution was added to terminate the reaction.

(2) Analysis conditions:

Instrument: Thermo Ultimate 3000 HPLC and MSQ Plus;
Chromatographic column: Welch Materials μltimate®XB-C18 (4.6 mm × 250 mm, 5 μm), Welch;
Analysis conditions: A solution (aqueous solution containing 0.1% TFA), solution B (acetonitrile solution containing 0.1% TFA);
Gradient: 0 to 70 min, B from 3 to 70%;
LC detection wavelength: 214 nm;
Ion source: ESI;
Ion type: positive ion;
Cone voltage: 50V;
Scanning range: 300 to 2000 Da;
Scan time: 1S;
Post-column flow splittered to MS: about 0.3ml/min.
100μl of the sample was injected, and the chromatogram was recorded.

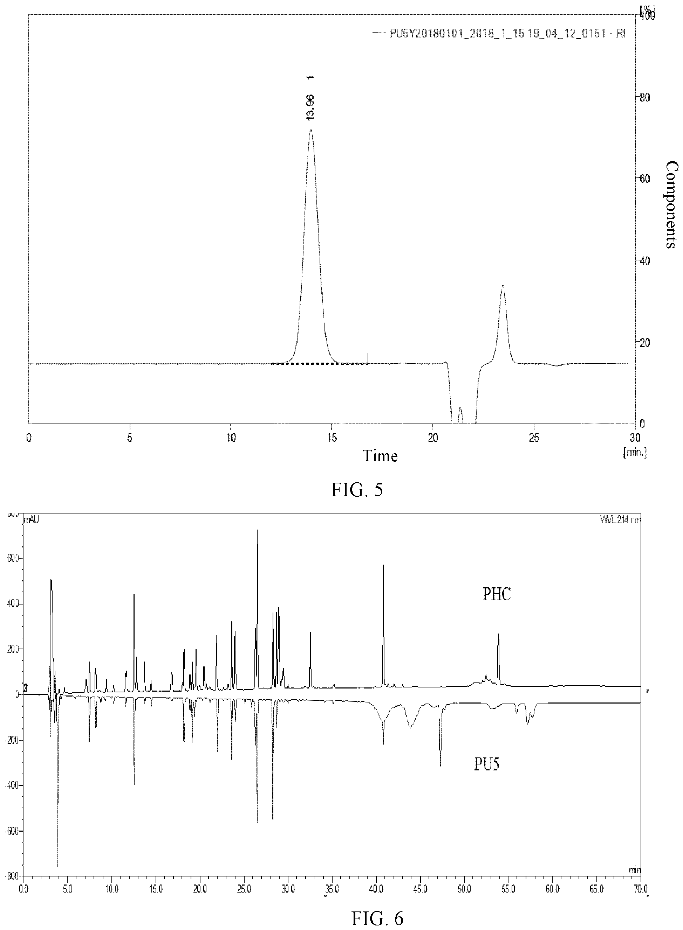

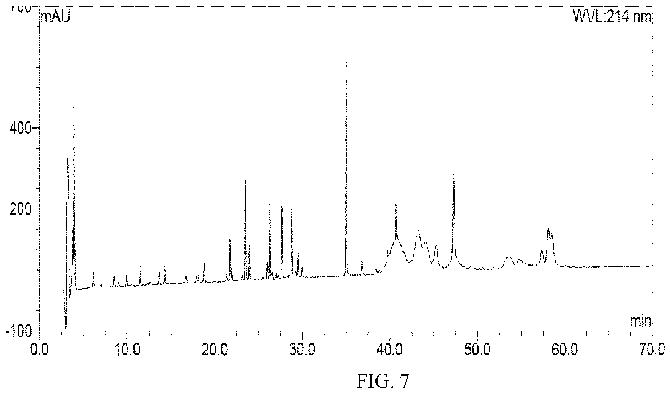

(3) Results processing:
The chromatograms (peptide maps) of urate oxidase and pegylated urate oxidase were compared, and the relative proportion of area reduction of the differential peptide fragments.
(4) The experimental results are shown in Table 2 to Table 5, and FIG. 6 to FIG. 7.

Table 2 List of peptide fragments of PHC after digestion with Lys-C

| Peptide fragment | Digestion site | Sequence | Theoretical molecular weight (Da) | Measured molecular weight |
| --- | --- | --- | --- | --- |
| T1 | 3 | TYK | 410.47 | 410.2 |
| T2 | 4 | K | 146.189 | / |
| T3 | 17 | NDEVEFVRTGYGK | 1513.627 | / |
| T2+T3 | | KNDEVEFVRTGYGK | 1641.089 | 1642.2 |
| T4 | 21 | DMIK | 505.63 | 505.4 |
| T5 | 30 | VLHIQRDGK | 1065.241 | 1065 |
| T6 | 35 | YHSIK | 646.744 | 646.5 |
| T7 | 48 | EVATTVQLTLSSK | 1376.57 | / |
| T8 | 49 | K | 146.189 | / |

(continued)

| Peptide fragment | Digestion site | Sequence | Theoretical molecular weight (Da) | Measured molecular weight |
|---|---|---|---|---|
| T9 | 66 | DYLHGDNSDVIPTDTIK | 1903.032 | 1903 |
| T10 | 74 | NTVNVLAK | 858.005 | 857.7 |
| T11 | 76 | FK | 293.366 | 293.1 |
| T12 | 79 | GIK | 316.401 | 316.2 |
| T13 | 97 | SIETFAVTICEHFLSSFK | 2059.364 | 2059 |
| T14 | 112 | HVIRAQVYVEEVPWK | 1853.154 | 1852.8 |
| T15 | 116 | RFEK | 578.669 | 578.4 |
| T16 | 120 | NGVK | 416.478 | 417.2 |
| T17 | 152 | HVHAFIYTPTGTHFCEVEQIRNGPPVIHSGIK | 3586.088 | 3586.2 |
| T18 | 155 | DLK | 374.437 | 374.1 |
| T19 | 158 | VLK | 358.481 | 358.2 |
| T20 | 169 | TTQSGFEGFIK | 1214.34 | 1213.8 |
| T21 | 179 | DQFTTLPEVK | 1177.32 | 1176.8 |
| T22 | 190 | DRCFATQVYCK | 1333.543 | 1333.2 |
| T23 | 215 | WRYHQGRDVDFEATWDTVRSIVLQK | 3106.45 | 3106.5 |
| T24 | 222 | FAGPYDK | 796.878 | 796.5 |
| T25 | 231 | GEYSPSVQK | 994.069 | 993.7 |
| T26 | 266 | TLYDIQVLTLGQVPEIEDMEISLPNIHYLNIDMSK | 4046.66 | 4046.1 |
| T27 | 272 | MGLINK | 674.856 | / |
| T28 | 285 | EEVLLPLDNPYGK | 1486.685 | 1486.6 |
| T27+T2 8 | | MGLINK EEVLLPLDNPYGK | 2143.54 | 2143.2 |
| T29 | 291 | ITGTVK | 617.743 | 617.4 |
| T30 | 293 | RK | 302.377 | / |
| T31 | 298 | LSSRL | 574.678 | 574.4 |

Table 3 List of peptide fragments of PHC after double-enzyme digestion with Lys-C and trypsin

| Peptide fragment | Sequence position | Sequence | Theoretical relative molecular weight [Da] | Measured molecular weight |
|---|---|---|---|---|
| T1 | 1-3 | TYK | 410.470 | 410.3 |
| T2 | 4 | K | 146.189 | / |
| T3 | 5-12 | NDEVEFVR | 1007.068 | / |
| T2+3 | 4-12 | KNDEVEFVR | | 1135.4 |
| T4 | 13-17 | TGYGK | 524.574 | 524.5 |
| T5 | 18-21 | DMIK | 505.630 | 505.5 |
| T6 | 22-27 | VLHIQR | 764.926 | 764.8 |
| T7 | 28-30 | DGK | 318.330 | / |
| T8 | 31-35 | YHSIK | 646.744 | 646.7 |
| T9 | 36-48 | EVATTVQLTLSSK | 1376.570 | / |
| T10 | 49 | K | 146.189 | / |
| T11 | 50-66 | DYLHGDNSDVIPTDTIK | 1903.032 | 1903.4 |
| T12 | 67-74 | NTVNVLAK | 858.005 | 857.9 |
| T13 | 75-76 | FK | 293.366 | 293.1 |
| T14 | 77-79 | GIK | 316.401 | / |
| T15 | 80-97 | SIETFAVTICEHFLSSFK | 2059.364 | 2059.6 |
| T16 | 98-101 | HVIR | 523.636 | 523.6 |
| T17 | 102-112 | AQVYVEEVPWK | 1347.534 | 1347.4 |
| T18 | 113 | R | 174.203 | / |
| T19 | 114-116 | FEK | 422.481 | / |
| T18+19 | 113-116 | RFEK | 578.684 | 578.6 |
| T20 | 117-120 | NGVK | 416.478 | 417.1 |
| T21 | 121-141 | HVHAFIYTPTGTHFCEVEQIR | 2485.802 | 2486.8 |
| T22 | 142-152 | NGPPVIHSGIK | 1118.301 | 1118.8 |
| T21+22 | 121-152 | | 3586.103 | 3587.7 |
| T23 | 153-155 | DLK | 374.437 | / |
| T24 | 156-158 | VLK | 358.481 | 358.3 |
| T25 | 159-169 | TTQSGFEGFIK | 1214.340 | 1214.2 |
| T26 | 170-179 | DQFTTLPEVK | 1177.320 | 1177.2 |
| T27 | 181-181 | DR | 289.291 | / |
| T28 | 182-190 | CFATQVYCK | 1062.267 | / |
| T27+28 | 181-190 | | 1333.558 | 1333.6 |
| T29 | 191-192 | WR | 360.416 | 360.1 |
| T30 | 193-197 | YHQGR | 659.702 | 659.6 |
| T31 | 198-209 | DVDFEATWDTVR | 1453.528 | 1453.6 |

(continued)

| Peptide fragment | Sequence position | Sequence | Theoretical relative molecular weight [Da] | Measured molecular weight |
|---|---|---|---|---|
| T32 | 210-215 | SIVLQK | 686.850 | 686.8 |
| T33 | 216-222 | FAGPYDK | 796.878 | 796.8 |
| T34 | 223-231 | GEYSPSVQK | 994.069 | 994.1 |
| T35 | 262-266 | TLYDIQVLTLGQVPEIEDM EISLPNIHYLNIDMSK | 4046.660 | 4047 |
| T36 | 267-272 | MGLINK | 674.856 | 674.7 |
| T37 | 273-285 | EEVLLPLDNPYGK | 1486.685 | 1486.7 |
| T36+37 | | | 2143.541 | 2143.6 |
| T38 | 286-291 | ITGTVK | 617.743 | 617.7 |
| T39 | 292 | R | 174.203 | / |
| T40 | 293 | K | 146.189 | / |
| T41 | 294-297 | LSSR | 461.519 | 461.5 |
| T42 | 298 | L | 131.175 | / |

The calculation method of the reduction percentage of peak area of PU5 peptide fragments is as follows:

The following formula can be used to calculate the corresponding peak areas of PU5 peptide fragments at the concentration of PU5 same as the concentration of PHC:

$$A_1 = A_0 \times t$$

where $A_1$ is a peak area of PU5 peptide fragments converted with two internal reference peptides, $A_0$ is a measured peak area of peptide fragments of a PU5 peptide map, and t is an average value of a peak area ratio of T30 internal reference peptide fragment in the PHC peptide map to T30 internal reference peptide fragment in the PU5 peptide map and a peak area ratio of T31 internal reference peptide fragment in the PHC peptide map to T31 internal reference peptide fragment in the PU5 peptide map, that is, t is 0.588.

Table 4 Comparison of PHC and PU5 internal reference peptide fragments

| Peptide fragment No. | Sequence | PHC peptide map | | PU5 peptide map | | Peak area ratio of PHC to PU5 | |
|---|---|---|---|---|---|---|---|
| | | Retention time | Peak area | Retention time | Peak area | Value | Mean |
| T30 | YHQGR | 7.5 | 13.4 | 7.467 | 22.9 | 0.585 | 0.588 |
| T31 | DVDFEA TWDTV R | 28.31 | 35.5 | 28.28 | 60.1 | 0.591 | |

With the peak area of peptide fragment converted with the internal reference and the peak area of PHC peptide map, the relative percentage of the reduction of the peak area of a certain peptide fragment in the PU5 peptide map can be calculated based on the following equation:

$$P(\%) = (A_2 - A_1)/A_2 \times 100\%$$

where $A_2$ is a peak area of a certain peptide fragment in the PHC peptide map, and $A_1$ is a peak area of this peptide fragment in PU5 converted with internal reference.

Table 5 Summary results of peptide fragments with reduced peak area in the peptide map of PU5 digested with double enzymes

| Peptide fragment position | Peptide fragment sequence | Relative proportion of reduced peak area of peptide fragment |
|---|---|---|
| 1-3 | TYK | 100.00% |
| 4-12 | KNDEVEFVR | 94.07% |
| 31-35 | YHSIK | 100.00% |
| 75-76 | FK | 82.27% |
| 80-97 | SIETFAVTICEHFLSSFK | 100.00% |
| 102-112 | AQVYVEEVPWK | 100.00% |
| 113-116 | RFEK | 100.00% |
| 117-120 | NGVK | 100.00% |
| 121-152 | HVHAFIYTPTGTHFCEVEQIRN GPPVIHSGIK | 100.00% |
| 193-197 | YHQGR | Internal reference peptide fragment |
| 198-209 | DVDFEATWDTVR | Internal reference peptide fragment |
| 216-222 | FAGPYDK | 91.37% |
| 223-231 | GEYSPSVQK | 86.40% |
| 232-266 | TLYDIQVLTLGQVPEIEDMEISL PNIHYLNIDMSK | 100.00% |
| 273-285 | EEVLLPLDNPYGK | 100.00% |

Based on the analysis of the protein sequence (SEQ ID NO: 1) of the present example, the potential sites for urate oxidase modification include 31 sites, including $T^1$, $K^3$, $K^4$, $K^{17}$, $K^{21}$, $K^{30}$, $K^{35}$, $K^{48}$, $K^{49}$, $K^{66}$, $K^{74}$, $K^{76}$, $K^{79}$, $K^{97}$, $K^{112}$, $K^{116}$, $K^{120}$, $K^{152}$, $K^{155}$, $K^{158}$, $K^{169}$, $K^{179}$, $K^{190}$, $K^{213}$, $K^{222}$, $K^{231}$, $K^{266}$, $K^{272}$, $K^{283}$, $K^{291}$, and $K^{293}$.

Based on the analysis of the modification sites of polyethylene glycol-modified urate oxidase obtained in Example 2, as shown in Table 2, Table 3, Table 4, Table 5 and FIG. 6, the sites with 90% or more disappearance of the peptide fragments after PU5 digestion include $K^3$, $K^4$, $K^{35}$, $K^{97}$, $K^{112}$, $K^{116}$, $K^{120}$, $K^{152}$, $K^{222}$, $K^{266}$, and $K^{285}$, the sites with 80% to 90% disappearance of the peptide fragments after PU5 digestion include $K^{76}$ and $K^{231}$. In PU5, these sites are all modified.

Moreover, the inventors found that the polyethylene glycol-modified urate oxidase of the present disclosure has more modification sites than the marketed drug, and has significant differences. For example, through single enzyme digestion, it was found that, the disappearance ratio of the peptide fragments where the four sites $K^{30}$, $K^{35}$, $K^{222}$, and $K^{231}$ are located in the polyethylene glycol-modified urate oxidase of the present disclosure is more than 80%. However, by analyzing the marketed analogous drug Krystexx (pegloticase) with the method, the peptide fragments where these four sites are located hardly disappeared, that is, the modification rate of the marketed analogous drug at the four sites $K^{30}$, $K^{35}$, $K^{222}$, and $K^{231}$ is much lower than that of the polyethylene glycol-modified urate oxidase of the present disclosure. In addition, the polyethylene glycol-modified urate oxidase of the present disclosure has significantly lower immunogenicity than the marketed drug, which may be related to the differences in the number of modification sites and the modification sites according to Applicant's speculation. Due to the differences in the modification sites and modification degree, the protection to the immunogenic site of the enzyme and the exposure of the active center of the enzyme in the body are both different. The above-mentioned differences may cause the difference in the biological properties of different modified enzymes in the body.

Since urate oxidase is a protein preparation and different chemical modifications will have different effects on its stability, it is necessary to use different excipients, pH, and the like for specific urate oxidase active ingredient to prepare the urate oxidase preparation when preparing the preparation. The following description will describe the preparation of a highly stable and biologically active urate oxidase preparation with the polyethylene glycol-modified urate oxidase of the present disclosure.

**Example 5: Formulation screening**

With the polyethylene glycol-modified urate oxidase PU5 mentioned in the above examples as an active ingredient, the components of the polyethylene glycol-modified uricase preparation were screened to obtain a polyethylene glycol uricase preparation with high uricase activity and high stability. The screening includes: excipient formulation screening and stabilizer screening.

1. Excipient screening

Excipients for preparation formulation include: carbonate, phosphate, hydrochloride, and citrate.
Using the above buffer excipients and the active ingredient to prepare the polyethylene glycol-modified urate oxidase preparation. Under the conditions of the same concentration range of 10 to 50 mmol/L and the same pH (pH 7 to 9) of excipients, the stability of the polyethylene glycol-modified urate oxidase was basically the same, but the concentration ratio of different excipients and the difficulty degree of osmotic pressure control in the preparation process was different, in which control of osmotic pressure and pH was easier for phosphate and hydrochloride than for carbonate and citrate. Through buffer screening, it was found that when phosphate and hydrochloride were used as excipients, the concentration range of phosphate was 10 to 50 mmol/L, and the concentration range of hydrochloride was 100 to 200 mmol/L, which could meet the technological requirements of preparation. Among them, a preparation with 15 mmol/L sodium dihydrogen phosphate/disodium hydrogen phosphate, 0.136 mol/L sodium chloride, and pH 7.4 had relatively good stability, and was used as a basic formulation for further screening.

2. Stabilizer screening experiment

Mannitol, glycerol, and Tween-80 were added in the basic preparation formulation. Whether it was necessary to add corresponding stabilizers in this preparation formulation was determined through a stability study.

2.1 Primary screening experiment of stabilizer

The basic formulation was used as a control. Stabilizers such as 4% mannitol, 2% glycerol, and 0.04% Tween-80 were added respectively to the basic formulation. The sample numbers were recorded as sample 1 to sample 4, respectively. Samples 1 to 4 were placed at 45°C for 7 days, and the particle size was measured to study the effect of stabilizers on the stability of PU5 preparation.
It can be seen from the experimental results that the particle size of the formulations added with glycerol and mannitol (19.44 and 20.51, respectively) was larger than that of the basic preparation formulation (18.57), and the particle size of the preparation formulation added with Tween-80 (18.10) was slightly smaller than that of the basic preparation formulation. It was considered that the addition of Tween-80 in the preparation formulation can prevent the increase of the particle size of PU5.

2.2 Stabilizer re-screening experiment

It can be seen from the preliminary screening experiment of the stabilizer that adding Tween-80 into the preparation formulation can prevent the increase of particle size of PU5, which is further confirmed by the stability. Two samples, sample 1 and sample 4, which were preliminarily selected and investigated at 45°C for 7 days, were placed into the investigation box at 45°C for additional 15 days. Samples were taken to detect the particle size, and the effect of Tween-80 stabilizer on the stability of PU5 preparation was studied.

(1) See Table 6 for results of particle size detection

Table 6 Summary of particle size results in stabilizer re-screening

| Sample Name | Treatment mode | Average particle size (nm) | Peak particle size (nm) | PDI (polydispersity index) | Percentage (%) of particle size >20 nm |
|---|---|---|---|---|---|
| No. 1 | Control | 18.46 | 20.51 | 0.108 | 4.0 |
| | 45°C for 7 days | 18.57 | 20.81 | 0.123 | 3.2 |
| | 45°C for 22 days | 18.14 | 20.59 | 0.111 | 2.4 |
| No. 4 | Control | 17.51 | 18.98 | 0.073 | 3.7 |
| | 45°C for 7 days | 18.10 | 19.91 | 0.089 | 3.7 |
| | 45°C for 22 days | 18.47 | 20.87 | 0.119 | 2.9 |
| Notes: control means being placed at a condition of 2 to 8°C. | | | | | |

(2) Conclusion

Data analysis from the investigation at 45°C for 7 days and 22 days vs the control group: when 0.04% Tween-80 was added, the PDI of the sample was significantly smaller and the sample was more uniform within 7 days at 45°C; when the detection was conducted on day 22 at 45°C, adding Tween-80 or not had no effect on the PDI, indicating that adding Tween-80 at high temperature had no significance in preventing or delaying the increase of particle size of PU5.

2.3 Third stabilizer screening experiment

In order to finally determine whether to add Tween-80 in the preparation formulation, two formulations, i.e., a formulation with Tween-80 and a formulation without Tween-80, were placed at 25°C for 1 month and 37°C for 1 month for investigation, respectively. Samples were taken to determine the particle size, high and low molecular weight protein contents, pH, and specific enzyme activity. The results are shown in Table 7.

Table 7 Results of accelerated stability study

| Investigation index | | Content (mg/ml) | High molecular weight protein content | Low molecular weight protein content | Specific enzyme activity (U/ mg) | Average particle size (nm) |
|---|---|---|---|---|---|---|
| Without adding Tween-80 | Before Investigation | 6.15 | 0.31% | none detected | 10.45 | / |
| | 25°C for 1 month | 5.98 | 0.17% | none detected | 9.60 | 17.93 |
| | 37°C for 1 month | 5.78 | 0.14% | 9.20% | 9.60 | 17.78 |
| Adding Tween-80 | Before Investigation | 6.46 | 0.37% | none detected | 9.07 | / |
| | 25°C for 1 month | 6.04 | 0.18% | none detected | 8.95 | 17.62 |
| | 37°C for 1 month | 6.00 | 0.20% | 9.90% | 8.75 | 17.32 |

It can be seen from Table 7 that the accelerated stability investigation of PU5 preparations with and without the addition of Tween-80 showed that the high and low molecular weight protein contents, enzyme activity, and particle size change behavior of PU5 preparations with and without the addition of Tween-80 were basically consistent.

After many times of investigations, there was no significant difference in the high and low molecular weight protein contents and particle size before and after adding Tween-80, so it was determined not to add the stabilizer in the preparation formulation.

**Example 6: Preparation pH screening**

1. pH range optimization

(1) Research method

The pH of the preparation formulation solution was adjusted to 7.8, 7.0, 7.4, 8.2, and 8.6 with buffer salts. The samples were placed at 2 to 8°C, 25 ± 2°C, and 37°C for stability study, and the contents of high molecular weight proteins and low molecular weight proteins and enzyme activity were determined on day 30. The polymerization or degradation of PU5 protein and the changes in enzyme activity were compared between the formulations.

(2) Detection results

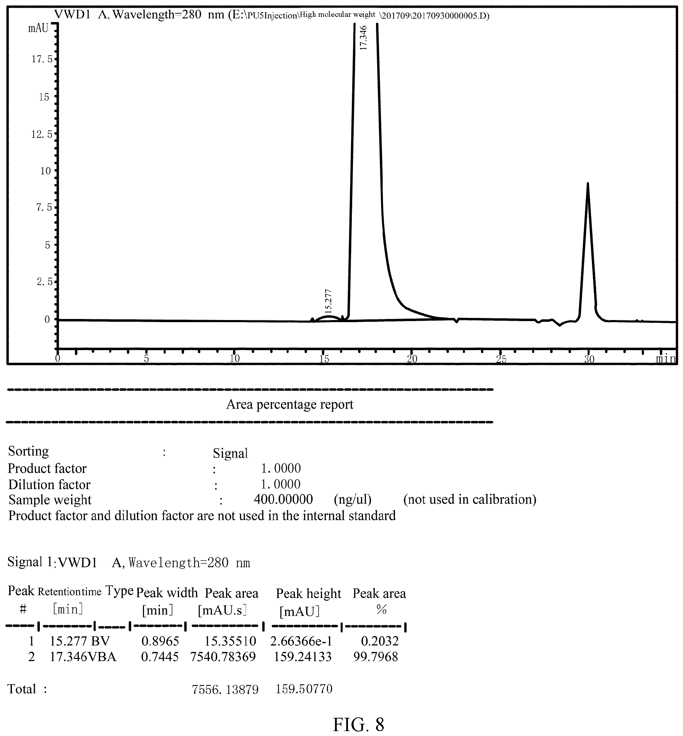

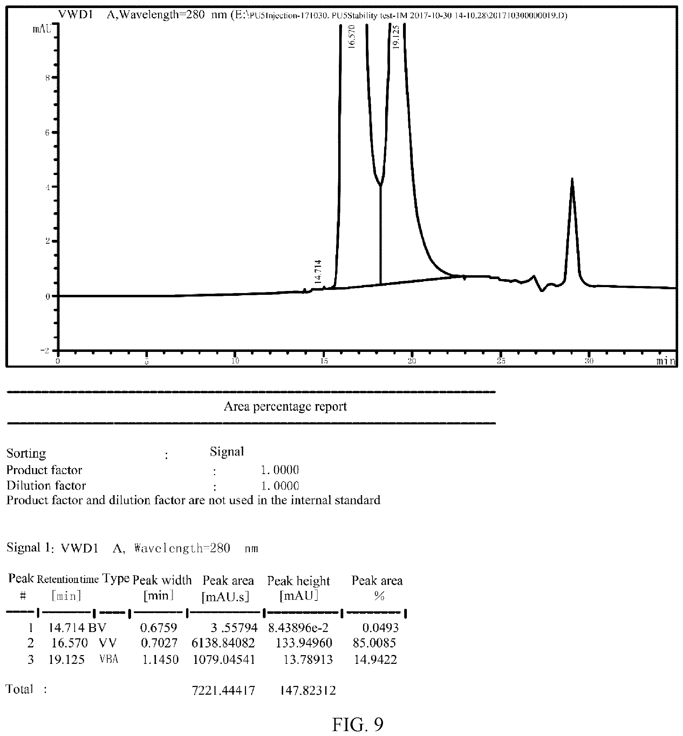

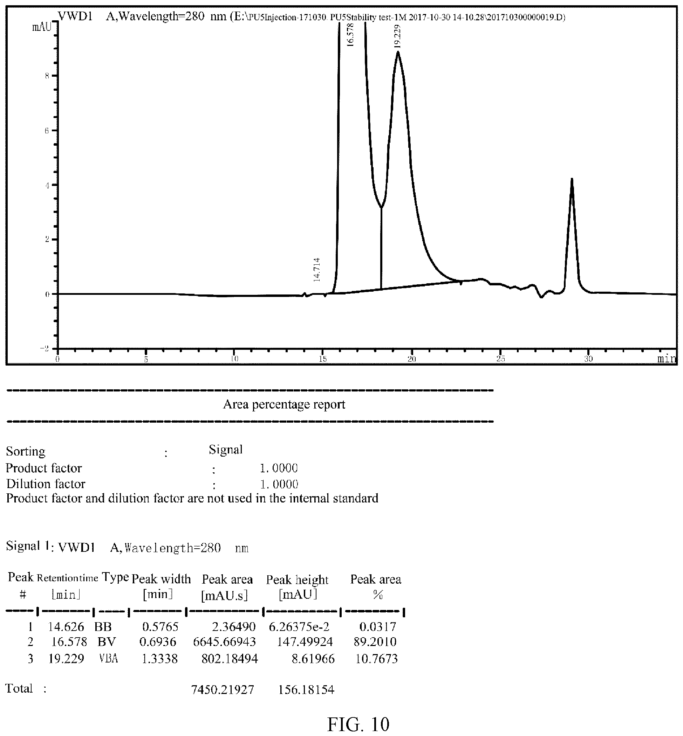

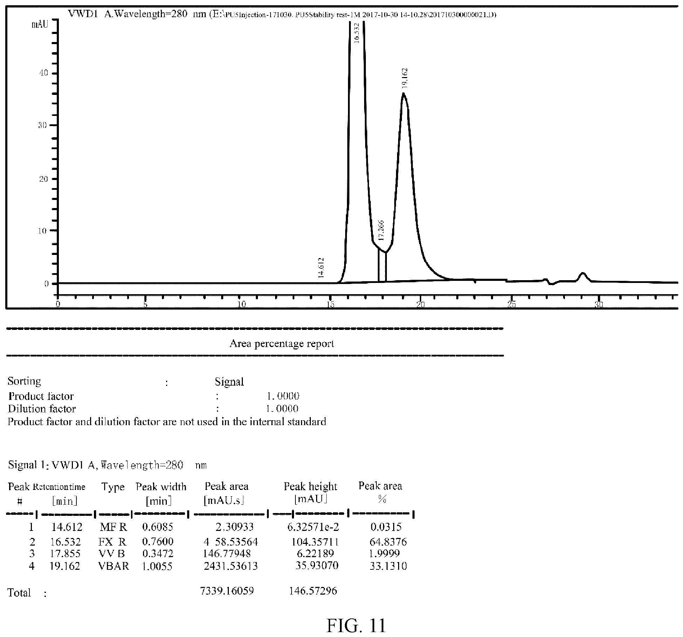

The results are shown in Table 8 and FIGS. 8 to 11.

Table 8 Summary of determination results of contents of high molecular weight proteins and low molecular weight proteins and enzyme activity (%)

| / | | Formulation 5 | Formulation 6 | Formulation 7 | Formulation 8 | Formulation 9 |
|---|---|---|---|---|---|---|
| | | | | | | |
| | | 7.8 | 8.2 | 7.4 | 7.0 | 8.6 |
| High weight molecule content | Day 0 | 0.56% | 0.82% | 0.67% | 0.33% | 0.22% |
| | 2 to 8°C for 30 days | 0.19% | 0.16% | 0.19% | 0.31% | 0.26% |
| | 25±2°C for 30 days | 0.14% | 0.12% | 0.15% | 0.21% | 0.13% |
| | 37°C for 30 days | 0.04% | 0.03% | Not detected | 0.17% | 0.05% |
| Low weight molecule content | Day 0 | <1.0% | <1.0% | <1.0% | 1.3% | <1.0% |
| | 2 to 8°C for 30 days | <1.0% | <1.0% | <1.0% | 2.3% | 1.4% |
| | 25±2°C for 30 days | <1.0% | <1.0% | <1.0% | 4.8% | 1.7% |
| | 37°C for 30 days | 14.7% | 10.5% | 23.4% | 27.1% | 19.6% |
| Specific enzyme activity (U/ mg) | Day 0 | 9.77 | 10.10 | 9.98 | 9.76 | 8.13 |
| | 25±2°C for 30 days | 9.71 | 9.58 | 9.36 | 8.40 | 9.09 |
| | 37°C for 30 days | 8.95 | 9.95 | 8.20 | 8.11 | 8.50 |
| Notes: "long term" means 2 to 8°C, "accelerated" means 25 ± 2°C, and "high temperature" means 37°C. | | | | | | |

It can be seen from FIGS. 8 to 11 and Table 8 that, after investigation at 2 to 8°C and 25 ± 2°C for 30 days, the content of high molecular weight proteins in Formulation 5 (pH 7.8), Formulation 6 (pH 8.2), and Formulation 7 (pH 7.4) showed a decreasing trend; the content of low molecular weight proteins showed a slight increase trend, of which Formulation 7> Formulation 5> Formulation 6. There was no significant change in enzyme activity among the three formulations. It was thus determined that the pH of the PU5 preparation was controlled between 7.4 and 8.2. After 30 days of high-temperature investigation at 37°C, there was no significant difference in the content of high molecular weight proteins among Formulations 5, 6, and 7, but the content of low molecular weight proteins had an increase, of which Formulation 7>Formulation 5>Formulation 6. The pH midpoint of the PU5 preparation was thus determined to be 7.8.

In summary, it was determined that the pH of PU5 injection preparation was in a range of 7.4 to 8.2.

The polyethylene glycol-modified urate oxidase of the present disclosure is significantly less immunogenic than the marketed drug, which may be related to the difference in the number of modified sites and modified sites according to the inventors' speculation. Due to the differences in the modification sites and modification degree, the protection to the immunogenic site of the enzyme and the exposure of the active center of the enzyme in the body are both different. The above differences may cause the difference in the biological properties of different modified enzymes in the body.

. The in vivo drug evaluation of the polyethylene glycol-modified urate oxidase preparation (PU5 preparation) of the present disclosure in animals will be described in detail below. The pegloticase used in the experiment refers to the analogous drug that has been marketed with a batch number 5085B.

**Example 7 Study on in vivo pharmacodynamics of polyethylene glycol-modified urate oxidase preparation**

7.1 In vivo efficacy evaluation of polyethylene glycol-modified urate oxidase in model rats

A chronic hyperuricemia rat model was induced by potassium oxazinate drinking water in combination with high uric acid feed, to evaluate the therapeutic effect of polyethylene glycol-modified urate oxidase (PU5) on chronic hyperuricemia in rats.

40 model rats were selected and randomly divided into 4 groups, i.e., a model group, a low-dose pegylated uricase administration group (0.3 mg/kg), a medium-dose pegylated uricase administration group (1.0 mg/kg), and a high-dose pegylated uricase administration group (3.0 mg/kg), 10 rats in each group; and additionally, 10 normal SD rats were selected as the blank control group. The model-establishing experiment was conducted for 5 consecutive weeks, and intramuscular administration was started 1 week after the start of the model establishing. The administration was performed and continued once a week for 4 consecutive weeks. The levels of serum uric acid, serum urea nitrogen, and serum creatinine of rats before the administration and 7 days after each administration were detected, and the histological changes of rat kidneys were observed after the experiment.

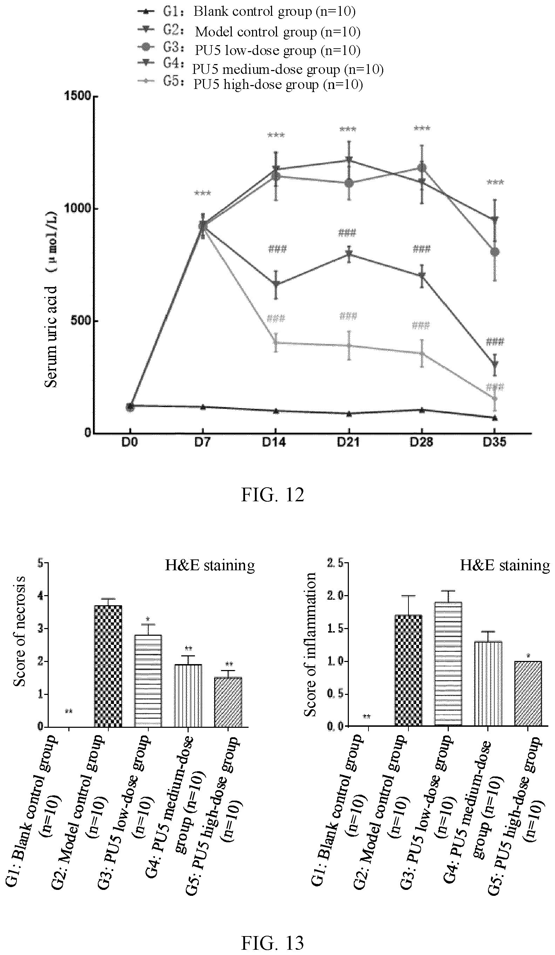

The results in FIG. 12 indicate that on days 7, 14, 21, 28, and 35 after the start of the model establishing, compared with the blank control group, the serum uric acid level of the model control group increased significantly, and the serum urea nitrogen, creatinine and uric acid of the rats in the model group were 2.73 times, 2.40 times and 7.83 times these in the blank group, respectively. From the perspective of kidney pathology (as shown in FIG. 13), the scores of renal tubule dilatation, necrosis, inflammation, and fibrosis of the model control group increased significantly, and the quantity of urate crystals also increased significantly. Both the medium and high doses of the test substance, pegylated uricase, significantly reduced the serum uric acid level, which exhibited a dose-dependent relationship. During the period from day 14 to day 35, the average serum uric acid level of the medium-dose group was maintained at 303.80 to 660.60 $\mu$mol/L, and the average serum uric acid level of the high-dose group was maintained at 153.70 to 403.40 $\mu$mol/L. Compared with the model group, the serum uric acid in the medium-dose group was reduced by 34.46% to 67.94%; the serum uric acid in the high-dose group was reduced by 65.67% to 83.78%. Compared with the model control group, each pegylated uricase administration group had significant ameliorating effects on the renal tubule dilatation, renal necrosis and inflammation.

7.2 Evaluation of single administration of polyethylene glycol-modified urate oxidase in rats

36 SD rats (half females and half males) were randomly divided into 6 groups (see Table 9), namely, a marketed drug Pegloticase intravenous injection group, a Pegloticase intramuscular injection group, a polyethylene glycol-modified urate oxidase intravenous injection group, as well as low-dose, medium-dose and high-dose polyethylene glycol-modified urate oxidase intramuscular injection groups (0.5 mg/kg, 1.0 mg/kg, 2.0 mg/kg). The specific administration regimens and doses are shown in Table 9. PK and PD were detected by taking the blood from the jugular vein.

Table 6 Animal grouping and dose design

| Number | Groups | Administration route | Administration frequency | Administration dose (mg/kg) | Administration concentration (mg/ml) | Administration volume (ml/kg) | Number of animals | |
|---|---|---|---|---|---|---|---|---|
| | | | | | | | Male | Female |
| 1 | Pegloticase intravenous injection group | Intravenous injection | One time | 1.0 | 0.1 | 10.0 | 3 | 3 |
| 2 | Pegloticase intramuscular injection Group | Intramuscular injection | One time | 1.0 | 1.0 | 1.0 | 3 | 3 |
| 3 | PU5 preparation intravenous injection group | Intravenous injection | One time | 1.0 | 0.1 | 10.0 | 3 | 3 |
| 4 | Low-dose intramuscular injection group of PU5 preparation | Intramuscular injection | One time | 0.5 | 0.5 | 1.0 | 3 | 3 |
| 5 | Medium-dose intramuscular injection group of PU5 preparation | Intramuscular injection | One time | 1.0 | 1.0 | 1.0 | 3 | 3 |
| 6 | High-dose intramuscular injection group of PU5 preparation | Intramuscular injection | One time | 2.0 | 2.0 | 1.0 | 3 | 3 |

EP 4 335 454 A1

### 7.2.1. Pharmacokinetic comparison

All the SD rats, before the administration, each had a serum drug concentration level lower than the lower limit of quantification (LLOQ: 312.500 ng/mL), and after one single intramuscular injection of 0.5 mg/kg, 1.0 mg/kg, and 2.0 mg/kg of the drug , in a period from 0h to 168h (0 to 7 days), the serum drug concentration of the pegylated uricase injection (PU5 preparation) was dose-dependent and had an overall level increasing with the increase in the administered dose; and 168 hours later, the blood drug concentration of the pegloticase intramuscular administration group was lower than the lower limit of quantification, and the blood drug concentration of the PU5 preparation intramuscular administration group maintained for more than 240h.

After administration, for each group of 1.0 mg/kg Pegloticase intravenous injection and intramuscular injection groups, 1.0 mg/kg pegylated uricase intravenous injection group, as well as 0.5 mg/kg, 1.0 mg/kg, and 2.0 mg/kg pegylated uricase intramuscular injection groups, , a ratio of Cmax (C5min) of female SD rats to male SD rats was in the range of 0.75 to 0.99, a ratio of AUClast of female SD rats to male SD rats was in the range of 0.54 to 0.94, and a ratio of AUC0-∞ of female SD rats to male SD rats was in the range of 0.58 to 0.97. It can be seen that there is no significant gender difference in the exposure levels of Pegloticase and the pegylated uricase (PU5 preparation) injections in SD rats.

However, when the SD rats were administered with the same dose (1.0 mg/kg), AUClast of the marketed drug Pegloticase intravenous administration group was $426.48 \pm 65.34$, AUClast of the Pegloticase intramuscular injection group was $264.19 \pm 78.22$; and AUClast of the PU5 preparation injection intravenous administration group was $565.61 \pm 161.60$, the AUClast of the PU5 preparation intramuscular injection group was $337.86 \pm 227.34$. Under the same dose and administration mode, the AUClast of PU5 preparation was higher than that of the marketed drug Pegloticase.

When the SD rats were administered with the same dose (1.0 mg/kg), t1/2(h) of the marketed drug Pegloticase intravenous administration group was $49.51 \pm 8.12$, t1/2(h) of the Pegloticase intramuscular administration group was $55.21 \pm 13.50$, t1/2(h) of the PU5 preparation injection intravenous administration group was $86.12 \pm 33.82$, and the t1/2(h) of the PU5 preparation intramuscular administration group was $60.45 \pm 21.37$. Under the same dose and administration mode, the t1/2(h) of PU5 preparation injection was longer than that of the marketed drug Pegloticase.

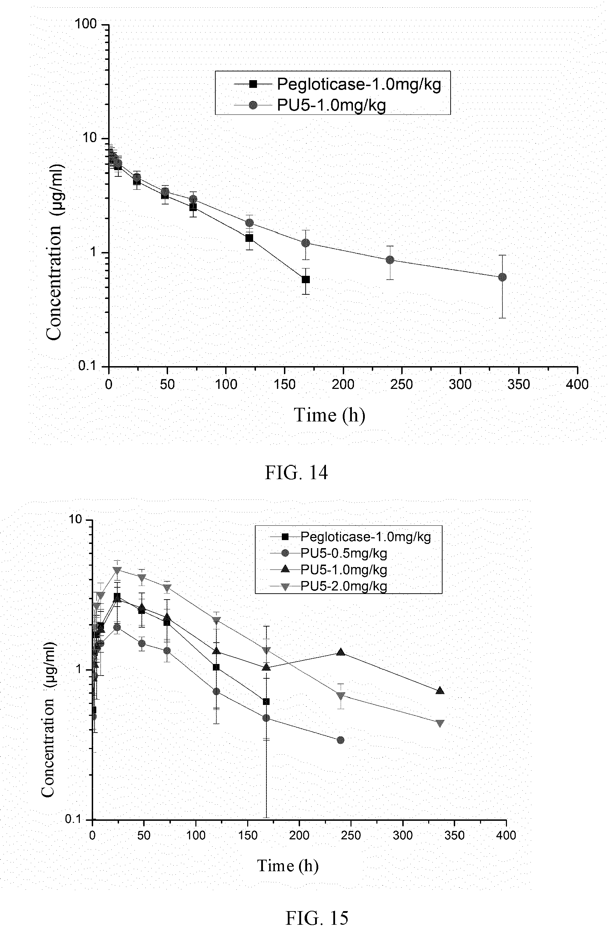

The above pharmacokinetic results are shown in Tables 10 to 15, and FIG. 14 to FIG. 16.

Table 10 Individual blood drug concentration data and statistical analysis data when the SD rats received a single intravenous injection of 1.0 mg/kg Pegloticase (unit: $\mu$g/mL)

| Sampling time (h) | Male | | | | | | Female | | | | | | Female + male | | |
|---|---|---|---|---|---|---|---|---|---|---|---|---|---|---|---|
| | 1M001 | 1M002 | 1M003 | N | Mean | SD | 1F001 | 1F002 | 1F003 | N | Mean | SD | N | Mean | SD |
| 0 | BLQ | BLQ | BLQ | 0 | / | / | BLQ | BLQ | BLQ | 0 | / | / | 0 | / | / |
| 0.08333 | 8.03 | 7.466 | 8.078 | 3 | 7.858 | 0.340 | 6.495 | 6.402 | 7.828 | 3 | 6.908 | 0.798 | 6 | 7.383 | 0.756 |
| 0.5 | 8.042 | 7.352 | 7.926 | 3 | 7.773 | 0.369 | 6.257 | 6.141 | 7.618 | 3 | 6.672 | 0.821 | 6 | 7.223 | 0.830 |
| 2 | 5.917 | 7.235 | 6.914 | 3 | 6.689 | 0.687 | 6.056 | 5.875 | 6.836 | 3 | 6.256 | 0.511 | 6 | 6.472 | 0.591 |
| 4 | 7.598 | 7.047 | 6.757 | 3 | 7.134 | 0.427 | 5.595 | 4.922 | 7.164 | 3 | 5.894 | 1.150 | 6 | 6.514 | 1.031 |
| 8 | 7.144 | 5.852 | 6.492 | 3 | 6.496 | 0.646 | 5.005 | 4.121 | 5.748 | 3 | 4.958 | 0.815 | 6 | 5.727 | 1.069 |
| 24 | 4.992 | 3.923 | 4.469 | 3 | 4.461 | 0.535 | 3.764 | 3.341 | 4.862 | 3 | 3.989 | 0.785 | 6 | 4.225 | 0.654 |
| 48 | 3.552 | 2.934 | 3.304 | 3 | 3.263 | 0.311 | 2.988 | 2.415 | 3.836 | 3 | 3.080 | 0.715 | 6 | 3.172 | 0.503 |
| 72 | 3.009 | 2.271 | 2.422 | 3 | 2.567 | 0.390 | 2.223 | 1.994 | 3.103 | 3 | 2.440 | 0.585 | 6 | 2.504 | 0.450 |
| 120 | 1.522 | 1.483 | 1.246 | 3 | 1.417 | 0.149 | 0.985 | 1.098 | 1.734 | 3 | 1.272 | 0.404 | 6 | 1.345 | 0.284 |
| 168 | 0.652 | 0.629 | 0.316 | 3 | 0.532 | 0.188 | 0.497 | 0.672 | 0.726 | 3 | 0.632 | 0.120 | 6 | 0.582 | 0.151 |
| 240 | BLQ | BLQ | BLQ | 0 | / | / | BLQ | BLQ | BLQ | 0 | / | / | 0 | / | / |
| 336 | BLQ | BLQ | BLQ | 0 | / | / | BLQ | BLQ | BLQ | 0 | / | / | 0 | / | / |
| Notes: "/" means no relevant information. | | | | | | | | | | | | | | | |

EP 4 335 454 A1

Table 11 Individual blood drug concentration data and statistical analysis data when the SD rats received a single intravenous injection of 1.0 mg/kg pegylated uricase (unit: $\mu$g/mL)

| Sampling time | Male | | | | | | Female | | | | | | Female + male | | |
|---|---|---|---|---|---|---|---|---|---|---|---|---|---|---|---|
| (h) | 3M001 | 3M002 | 3M003 | N | Mean | SD | 3F001 | 3F002 | 3F003 | N | Mean | SD | N | Mean | SD |
| 0 | BLQ | BLQ | BLQ | 0 | / | / | BLQ | BLQ | BLQ | 0 | / | / | 0 | / | / |
| 0.08333 | 7.364 | 9.941 | 7.74 | 3 | 8.348 | 1.392 | 7.236 | 5.991 | 6.657 | 3 | 6.628 | 0.623 | 6 | 7.488 | 1.348 |
| 0.5 | 7.316 | 9.469 | 7.693 | 3 | 8.159 | 1.150 | 7.051 | 5.513 | 6.36 | 3 | 6.308 | 0.770 | 6 | 7.234 | 1.340 |
| 2 | 7.742 | 9.084 | 7.338 | 3 | 8.055 | 0.914 | 6.063 | 5.522 | 6.44 | 3 | 6.008 | 0.461 | 6 | 7.032 | 1.294 |
| 4 | 7 | 8.837 | 6.997 | 3 | 7.611 | 1.061 | 6.508 | 5.735 | 6.288 | 3 | 6.177 | 0.398 | 6 | 6.894 | 1.064 |
| 8 | 6.628 | 7.43 | 6.61 | 3 | 6.889 | 0.468 | 5.387 | 4.85 | 5.52 | 3 | 5.252 | 0.355 | 6 | 6.071 | 0.971 |
| 24 | 4.672 | 5.628 | 4.746 | 3 | 5.015 | 0.532 | 4.291 | 3.919 | 4.129 | 3 | 4.113 | 0.187 | 6 | 4.564 | 0.609 |
| 48 | 3.307 | 4.264 | 3.497 | 3 | 3.689 | 0.507 | 3.406 | 3.042 | 3.014 | 3 | 3.154 | 0.219 | 6 | 3.422 | 0.456 |
| 72 | 2.933 | 3.762 | 3.124 | 3 | 3.273 | 0.434 | 2.859 | 2.596 | 2.319 | 3 | 2.591 | 0.270 | 6 | 2.932 | 0.494 |
| 120 | 1.986 | 2.279 | 1.989 | 3 | 2.085 | 0.168 | 1.604 | 1.617 | 1.454 | 3 | 1.558 | 0.091 | 6 | 1.822 | 0.313 |
| 168 | 1.268 | 1.742 | 1.391 | 3 | 1.467 | 0.246 | 1.187 | 1.031 | 0.699 | 3 | 0.972 | 0.249 | 6 | 1.220 | 0.350 |
| 240 | 0.67 | 1.19 | 0.734 | 3 | 0.865 | 0.284 | BLQ | BLQ | BLQ | 0 | / | / | 3 | 0.865 | 0.284 |
| 336 | BLQ | 0.853 | 0.368 | 2 | 0.611 | 0.343 | BLQ | BLQ | BLQ | 0 | / | / | 2 | 0.611 | 0.343 |
| Notes: "/" means no relevant information. | | | | | | | | | | | | | | | |

Table 12 Individual blood drug concentration data and statistical analysis data when the SD rats received a single intramuscular injection of 1.0 mg/kg Pegloticase (unit: $\mu$g/mL)

| Sampli ng time | Male | | | | | | Female | | | | | | Female + male | | |
|---|---|---|---|---|---|---|---|---|---|---|---|---|---|---|---|
| (h) | 2M001 | 2M002 | 2M003 | N | Mean | SD | 2F001 | 2F002 | 2F003 | N | Mean | SD | N | Mean | SD |
| 0 | BLQ | BLQ | BLQ | 0 | / | / | BLQ | BLQ | BLQ | 0 | / | / | 0 | / | / |
| 0.5 | 0.652 | BLQ | 0.581 | 2 | 0.617 | 0.050 | 0.388 | BLQ | BLQ | 1 | 0.388 | / | 3 | 0.540 | 0.137 |
| 2 | 1.337 | 1.249 | 1.62 | 3 | 1.402 | 0.194 | 1.172 | 1.135 | BLQ | 2 | 1.154 | 0.026 | 5 | 1.303 | 0.194 |
| 4 | 2.298 | 1.699 | 2.348 | 3 | 2.115 | 0.361 | 1.812 | 1.371 | 0.773 | 3 | 1.319 | 0.521 | 6 | 1.717 | 0.593 |
| 8 | 2.56 | 2.058 | 2.396 | 3 | 2.338 | 0.256 | 1.915 | 1.657 | 1.273 | 3 | 1.615 | 0.323 | 6 | 1.977 | 0.474 |
| 24 | 3.808 | 3.235 | 3.309 | 3 | 3.451 | 0.312 | 2.947 | 2.808 | 2.493 | 3 | 2.749 | 0.233 | 6 | 3.100 | 0.456 |
| 48 | 3.188 | 2.618 | 2.749 | 3 | 2.852 | 0.299 | 2.317 | 2.279 | 1.729 | 3 | 2.108 | 0.329 | 6 | 2.480 | 0.495 |
| 72 | 2.694 | 2.263 | 2.211 | 3 | 2.389 | 0.265 | 1.984 | 2.016 | 1.261 | 3 | 1.754 | 0.427 | 6 | 2.072 | 0.471 |
| 120 | 1.56 | 1.169 | 1.332 | 3 | 1.354 | 0.196 | 0.884 | 1.111 | 0.174 | 3 | 0.723 | 0.489 | 6 | 1.038 | 0.480 |
| 168 | BLQ | 0.341 | 0.869 | 2 | 0.605 | 0.373 | BLQ | 0.635 | BLQ | 1 | 0.635 | / | 3 | 0.615 | 0.265 |
| 240 | BLQ | BLQ | BLQ | 0 | / | / | BLQ | BLQ | BLQ | 0 | / | / | 0 | / | / |
| 336 | BLQ | BLQ | BLQ | 0 | / | / | BLQ | BLQ | BLQ | 0 | / | / | 0 | / | / |
| Notes: "/" means no relevant information. | | | | | | | | | | | | | | | |

Table 13 Individual blood drug concentration data and statistical analysis data when the SD rats received a single intramuscular injection of 1.0 mg/kg pegylated uricase injection (unit: $\mu$g/mL)

| Sampling time | Male | | | | | | Female | | | | | | Female + male | | |
|---|---|---|---|---|---|---|---|---|---|---|---|---|---|---|---|
| (h) | 5M001 | 5M002 | 5M003 | N | Mean | SD | 5F001 | 5F002 | 5F003 | N | Mean | SD | N | Mean | SD |
| 0 | BLQ | BLQ | BLQ | 0 | / | / | BLQ | BLQ | BLQ | 0 | / | / | 0 | / | / |
| 0.5 | BLQ | 1.421 | 0.328 | 2 | 0.875 | 0.773 | BLQ | BLQ | BLQ | 0 | / | / | 2 | 0.875 | 0.773 |
| 2 | BLQ | 2.295 | 0.923 | 2 | 1.609 | 0.970 | 0.593 | 0.905 | 0.674 | 3 | 0.724 | 0.162 | 5 | 1.078 | 0.695 |
| 4 | 0.729 | 2.897 | 1.648 | 3 | 1.758 | 1.088 | 1.356 | 1.222 | 0.762 | 3 | 1.113 | 0.312 | 6 | 1.436 | 0.798 |
| 8 | 1.305 | 3.628 | 2.054 | 3 | 2.329 | 1.186 | 1.559 | 1.249 | 1.266 | 3 | 1.358 | 0.174 | 6 | 1.844 | 0.926 |
| 24 | 2.408 | 4.617 | 3.069 | 3 | 3.365 | 1.134 | 3.01 | 2.339 | 2.216 | 3 | 2.522 | 0.427 | 6 | 2.943 | 0.895 |
| 48 | 2.068 | 3.877 | 2.4 | 3 | 2.782 | 0.963 | 2.739 | 2.298 | 2.189 | 3 | 2.409 | 0.291 | 6 | 2.595 | 0.668 |
| 72 | 1.76 | 3.606 | 2.027 | 3 | 2.464 | 0.998 | 2.385 | 1.761 | 1.863 | 3 | 2.003 | 0.335 | 6 | 2.234 | 0.712 |
| 120 | 1.042 | 2.9 | 1.107 | 3 | 1.683 | 1.054 | 1.169 | 0.811 | 0.926 | 3 | 0.969 | 0.183 | 6 | 1.326 | 0.782 |
| 168 | 0.479 | 2.419 | 0.631 | 3 | 1.176 | 1.079 | 0.595 | BLQ | BLQ | 1 | 0.595 | / | 4 | 1.031 | 0.928 |
| 240 | BLQ | 1.303 | BLQ | 1 | 1.303 | / | BLQ | BLQ | BLQ | 0 | / | / | 1 | 1.303 | / |
| 336 | BLQ | 0.719 | BLQ | 1 | 0.719 | / | BLQ | BLQ | BLQ | 0 | / | / | 1 | 0.719 | / |
| Notes: "/" means no relevant information. | | | | | | | | | | | | | | | |

Table 14 Average pharmacokinetic parameters of SD rats after a single intravenous injection of Pegloticase and pegylated uricase injections

| Dose | Gender | Paramet er | $t_{1/2}$ | C5 min | $AUC_{last}$ | $AUC_{0-\infty}$ | Vz | Cl | $MRT_{last}$ |
|---|---|---|---|---|---|---|---|---|---|
| (mg/kg) | | | (h) | ($\mu$g/mL) | (h*$\mu$g/mL) | (h*$\mu$g/mL) | (mL/kg) | (mL/h/ kg) | (h) |
| 1.0 (Pegloticas e) | Male | N | 3 | 3 | 3 | 3 | 3 | 3 | 3 |
| | | Mean | 45.70 | 7.86 | 448.57 | 484.58 | 136.90 | 2.08 | 52.36 |
| | | SD | 7.57 | 0.34 | 42.16 | 46.96 | 26.57 | 0.19 | 2.58 |
| | Female | N | 3 | 3 | 3 | 3 | 3 | 3 | 3 |
| | | Mean | 53.32 | 6.91 | 404.38 | 453.63 | 173.91 | 2.26 | 54.64 |
| | | SD | 7.98 | 0.80 | 86.21 | 90.21 | 43.64 | 0.40 | 2.62 |
| | Female + male | N | 6 | 6 | 6 | 6 | 6 | 6 | 6 |
| | | Mean | 49.51 | 7.39 | 426.48 | 469.11 | 155.40 | 2.17 | 53.50 |
| | | SD | 8.12 | 0.76 | 65.34 | 66.52 | 38.14 | 0.30 | 2.64 |
| 1.0 (PU5 preparation ) | Male | N | 3 | 3 | 3 | 3 | 3 | 3 | 3 |
| | | Mean | 105.16 | 8.35 | 692.29 | 794.77 | 186.76 | 1.31 | 90.87 |
| | | SD | 41.08 | 1.39 | 128.22 | 197.50 | 24.94 | 0.29 | 14.06 |
| | Female | N | 3 | 3 | 3 | 3 | 3 | 3 | 3 |
| | | Mean | 67.09 | 6.63 | 438.93 | 535.17 | 180.63 | 1.88 | 58.58 |
| | | SD | 9.24 | 0.62 | 26.51 | 59.13 | 11.64 | 0.21 | 2.76 |
| | Female + male | N | 6 | 6 | 6 | 6 | 6 | 6 | 6 |
| | | Mean | 86.12 | 7.49 | 565.61 | 664.97 | 183.70 | 1.59 | 74.73 |
| | | SD | 33.82 | 1.35 | 161.60 | 192.92 | 17.73 | 0.39 | 19.87 |

Table 15 Average pharmacokinetic parameters of SD rats after a single intramuscular injection of Peglocticase and pegylated uricase injections

| Dose (mg/kg) | Gender | Paramet er | $t_{1/2}$ (h) | $T_{max}$ (h) | $C_{max}$ ($\mu$g/mL) | $AUC_{last}$ (h*$\mu$g/mL) | $AUC_{0-\infty}$ (h*$\mu$g/mL) | Vz F (mL/kg) | Cl F (mL/h/kg) | $MRT_{last}$ (h) |
|---|---|---|---|---|---|---|---|---|---|---|
| 1.0 (Pegloticase) | Male | N | 3 | 3 | 3 | 3 | 3 | 3 | 3 | 3 |
| | | Mean | 58.31 | 24.00 | 3.45 | 318.23 | 405.13 | 203.75 | 2.54 | 60.57 |
| | | SD | 20.10 | 0.00 | 0.31 | 15.37 | 80.13 | 42.90 | 0.54 | 6.54 |
| | Female | N | 3 | 3 | 3 | 3 | 3 | 3 | 3 | 3 |
| | | Mean | 52.12 | 24.00 | 2.75 | 210.14 | 278.56 | 276.83 | 3.72 | 51.27 |
| | | SD | 4.78 | 0.00 | 0.23 | 79.35 | 60.90 | 50.57 | 0.91 | 15.28 |
| | Female + male | N | 6 | 6 | 6 | 6 | 6 | 6 | 6 | 6 |
| | | Mean | 55.21 | 24.00 | 3.10 | 264.19 | 341.85 | 240.29 | 3.13 | 55.92 |
| | | SD | 13.50 | 0.00 | 0.46 | 78.22 | 94.12 | 57.97 | 0.93 | 11.68 |
| 0.5 (PU5 preparation) | Male | N | 3 | 3 | 3 | 3 | 3 | 3 | 3 | 3 |
| | | Mean | 63.57 | 24.00 | 1.93 | 181.10 | 233.11 | 199.06 | 2.21 | 60.26 |
| | | SD | 18.68 | 0.00 | 0.26 | 79.19 | 48.71 | 56.50 | 0.45 | 23.89 |
| | Female | N | 3 | 3 | 3 | 3 | 3 | 3 | 3 | 3 |
| | | Mean | 48.20 | 24.00 | 1.91 | 170.63 | 205.87 | 167.09 | 2.56 | 55.67 |
| | | SD | 17.38 | 0.00 | 0.14 | 41.99 | 61.41 | 21.47 | 0.67 | 11.48 |
| | Female + male | N | 6 | 6 | 6 | 6 | 6 | 6 | 6 | 6 |
| | | Mean | 55.88 | 24.00 | 1.92 | 175.87 | 219.49 | 183.07 | 2.38 | 57.97 |
| | | SD | 18.20 | 0.00 | 0.19 | 56.98 | 51.77 | 42.05 | 0.54 | 16.95 |

(continued)

| Dose (mg/kg) | Gender | Parameter | $t_{1/2}$ (h) | $T_{max}$ (h) | $C_{max}$ (µg/mL) | $AUC_{last}$ (h*µg/mL) | $AUC_{0-\infty}$ (h*µg/mL) | Vz F (mL/kg) | Cl F (mL/h/kg) | $MRT_{last}$ (h) |
|---|---|---|---|---|---|---|---|---|---|---|
| 1.0 (PU5 preparation) | Male | N | 3 | 3 | 3 | 3 | 3 | 3 | 3 | 3 |
| | | Mean | 70.47 | 24.00 | 3.36 | 439.83 | 504.61 | 225.86 | 2.57 | 84.20 |
| | | SD | 28.55 | 0.00 | 1.13 | 307.66 | 344.91 | 54.21 | 1.32 | 31.10 |
| | Female | N | 3 | 3 | 3 | 3 | 3 | 3 | 3 | 3 |
| | | Mean | 50.44 | 24.00 | 2.52 | 235.90 | 293.04 | 252.46 | 3.46 | 58.28 |
| | | SD | 5.05 | 0.00 | 0.43 | 58.01 | 45.24 | 46.82 | 0.50 | 6.96 |
| | Female + male | N | 6 | 6 | 6 | 6 | 6 | 6 | 6 | 6 |
| | | Mean | 60.45 | 24.00 | 2.94 | 337.86 | 398.83 | 239.16 | 3.02 | 71.24 |
| | | SD | 21.37 | 0.00 | 0.89 | 227.34 | 248.66 | 47.59 | 1.02 | 24.65 |

| Dose (mg/kg) | Gender | Parameter | $t_{1/2}$ (h) | $T_{max}$ (h) | $C_{max}$ (µg/mL) | $AUC_{last}$ (h*µg/mL) | $AUC_{0-\infty}$ (h*µg/mL) | $Vz\_F$ (mL/kg) | $Cl\_F$ (mL/h/kg) | $MRT_{last}$ (h) |
|---|---|---|---|---|---|---|---|---|---|---|
| 2.0 (PU5 preparation) | Male | N | 3 | 3 | 3 | 3 | 3 | 3 | 3 | 3 |
| | | Mean | 66.65 | 24.00 | 4.84 | 590.58 | 649.31 | 292.61 | 3.10 | 85.42 |
| | | SD | 20.11 | 0.00 | 0.46 | 59.68 | 55.26 | 64.39 | 0.27 | 19.91 |
| | Female | N | 3 | 3 | 3 | 3 | 3 | 3 | 3 | 3 |
| | | Mean | 72.51 | 32.00 | 4.55 | 537.05 | 628.72 | 339.98 | 3.22 | 79.26 |
| | | SD | 15.56 | 13.86 | 0.91 | 124.85 | 78.17 | 100.19 | 0.42 | 9.60 |
| | Female + male | N | 6 | 6 | 6 | 6 | 6 | 6 | 6 | 6 |
| | | Mean | 69.58 | 28.00 | 4.70 | 563.81 | 639.01 | 316.30 | 3.16 | 82.34 |
| | | SD | 16.40 | 9.80 | 0.66 | 92.30 | 61.59 | 79.67 | 0.32 | 14.38 |

7.2.2. Comparison of in vivo efficacy (uric acid)

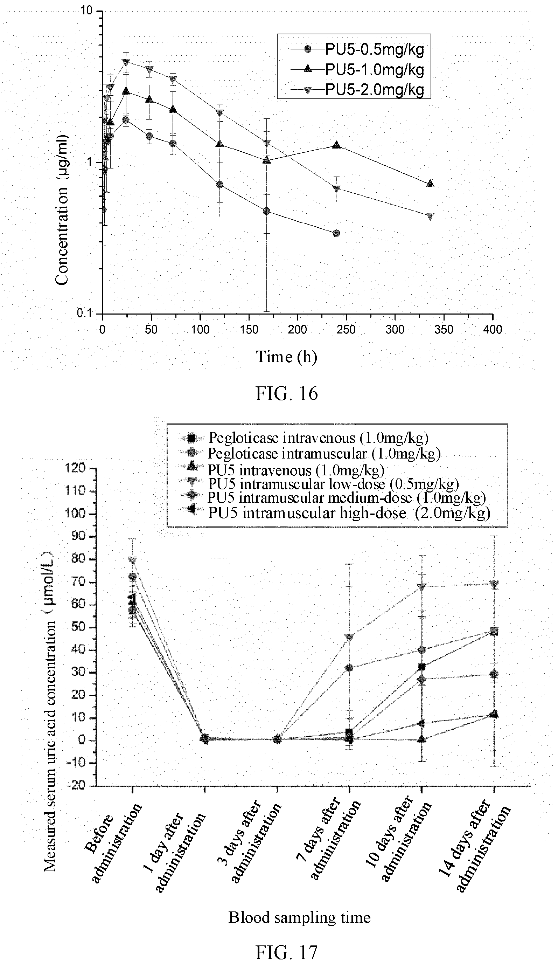

After a single intramuscular injection of 0.5 mg/kg, 1.0 mg/kg, 2.0 mg/kg pegylated uricase injection, the uric acid concentration maintained at a low level 1 day and 3 days after the administration, the uric acid level of each dose group began to recover 7 days after the administration, and the higher the dose, the longer the uric acid maintained a lower level in the body. Comparing the intravenous injection groups of the same dose, the PU5 preparation intravenous injection group maintained a low concentration level of serum uric acid for a longer time than the pegloticase intravenous injection group. Comparing the intramuscular injection groups of the same dose, the PU5 preparation intramuscular injection group maintained a low concentration level of serum uric acid for a longer time than the pegloticase intramuscular injection group. Comparing the groups of the same dose, the PU5 preparation intravenous or intramuscular injection group maintained a low concentration level of serum uric acid for a longer time than the pegloticase intravenous or intramuscular injection group, that is, the PU5 preparation maintained a low concentration level of uric acid in the body for a longer time than pegloticase in each case. The results are illustrated in FIG. 17.

7.3 Evaluation of multiple administrations of polyethylene glycol-modified urate oxidase in rats

In this experiment, 4 groups were set, i.e., a marketed drug Pegloticase intravenous injection group, a Pegloticase intramuscular injection group, a pegylated uricase (PU5) intravenous injection group, and a PU5 intramuscular injection group, each group included 8 rats, four of which were male and the other four of which were female, 32 SD rats in total. The Pegloticase and pegylated uricase intravenous injection groups adopted intravenous injection; and the Pegloticase and pegylated uricase intramuscular injection groups adopted intramuscular injection. The administration dose was 1.0 mg/kg, once a week, for 4 consecutive times.
The result analysis indicates that:
SD rats were injected intravenously/intramuscularly with 1.0 mg/kg Pegloticase and pegloticase injections for multiple times, The general condition of the rats had no abnormal changes related to the drugs.

7.3.1 Anti-PEG antibody detection

SD rats were administrated for 4 consecutive times. Before the first administration, no anti-PEG antibodies and no anti-PHC antibodies were detected in either individual animal; after the administration, no anti-PHC antibodies were detected in either animal, anti-PEG antibodies were detected in each group of the Pegloticase intravenous and intramuscular injection groups as well as the pegylated uricase intravenous and intramuscular injection groups, and the ratios of positive results were: 3/8, 1/8, 1/8, and 1/8, respectively. The PEG immunohistochemical examination revealed that the spleen, liver and kidney of the pegloticase intravenous and intramuscular injection groups showed weak positive expression of PEG; and no positive expression of PEG was found in the pegylated uricase intravenous and intramuscular injection groups. The results are shown in Table 13.
It can be seen from the above analysis that the antibodies produced by PU5 preparation and pegloticase are mainly antibodies against the PEG part, rather than antibodies against the urate oxidase part, indicating that both can effectively shield the immunogenic site of urate oxidase. The production of PEG antibodies may cause some side effects in vivo. According to the results in Table 16, the immunogenicity of the PU5 preparation of the present disclosure is lower than that of the commercially available product pgeloticase.
According to the results of PEG antibody and PEG immunohistochemistry, the intramuscular administration groups of both PU5 preparation and pegloticase are superior to the intravenous administration groups thereof. Among them, regarding the produced anti-PEG antibodies of the intravenous administration groups, the PU5 preparation is superior to pegloticase; and regarding the produced anti-PEG antibodies of the intramuscular administration groups, the PU5 preparation is superior to pegloticase.

Table 16 PEG immunohistochemistry positive expression results

| | | Incidence | | | | | | | |
|---|---|---|---|---|---|---|---|---|---|
| | | Pegloticase intravenous injection group | | Pegloticase intramuscular injection Group | | PU5 preparation intravenous injection group | | PU5 preparation intramuscular injection group | |
| Microscopic observation | | Male | Female | Male | Female | Male | Female | Male | Female |
| **Spleen** | Total inspection number: | 4 | 4 | 4 | 4 | 4 | 4 | 4 | 4 |
| --PEG, white pulp | 1 | 0 | 1 | 1 | 2 | 0 | 0 | 0 | 0 |
| | Total incidence number: | 0 | 1 | 1 | 2 | 0 | 0 | 0 | 0 |
| --PEG, red pulp | 1 | 0 | 1 | 0 | 0 | 0 | 0 | 0 | 0 |
| | Total incidence number: | 0 | 1 | 0 | 0 | 0 | 0 | 0 | 0 |
| **Liver** | Total inspection number: | 4 | 4 | 4 | 4 | 4 | 4 | 4 | 4 |
| --PEG, vascular endothelial cells/Kupffer cells | 1 | 4 | 4 | 1 | 3 | 0 | 0 | 0 | 0 |
| | Total incidence number: | 4 | 4 | 1 | 3 | 0 | 0 | 0 | 0 |
| **Kidney** | Total inspection number: | 4 | 4 | 4 | 4 | 4 | 4 | 4 | 4 |
| --PEG, renal tubules | 1 | 3 | 1 | 1 | 1 | 0 | 0 | 0 | 0 |
| | Total incidence number: | 3 | 1 | 1 | 1 | 0 | 0 | 0 | 0 |
| --PEG, vascular endothelial cells | 1 | 0 | 1 | 0 | 0 | 0 | 0 | 0 | 0 |
| | Total incidence number: | 0 | 1 | 0 | 0 | 0 | 0 | 0 | 0 |
| Positive grading: 1 = extremely weakly positive, 2 = weakly positive, 3 = moderately positive, 4 = strongly positive. | | | | | | | | | |

4.3.2 Pharmacokinetic tests

The SD rats that had received multiple intravenous and intramuscular injections of Pegloticase and pegylated uricase injections exhibited no significant gender difference in the main pharmacokinetic parameters between the groups.

After 4 consecutive administrations, these two drugs accumulated slightly in rats.

When the SD rats received multiple intravenous/intramuscular injection administrations with the same dose (1.0 mg/kg) of the marketed drug Pegloticase, the absolute bioavailability in the rats was 51.35% after the first administration; and the absolute bioavailability in the rats was 45.98% after the last administration. When the SD rats received multiple intravenous/intramuscular injection administrations with the same dose (1.0 mg/kg) of the pegylated uricase injection, the absolute bioavailability in the rats was 58.29% after the first administration; and the absolute bioavailability in the rats was 52.60% after the last administration.

4.3.3 Comparison of in vivo drug efficacy (uric acid)

SD rats were injected intravenously and intramuscularly with 1.0 mg/kg Pegloticase and 1.0 mg/kg pegylated uricase injection for 4 consecutive times (1 time/week), the serum uric acid concentration maintained at a low level after each administration; the serum uric acid concentration recovered 14 days after the last administration in the Pegloticase intramuscular injection group, and in the rest groups, the serum uric acid concentration recovered 18 days after the last administration. Compared with the same dose of the marketed drug Pegloticase, the maintaining time in the intravenous injection groups of these two drugs was basically consistent, and the maintaining time of the pegylated uricase intramuscular injection group was longer than that of the marketed drug intramuscular injection group. That is, for the intramuscular administration, the efficacy of PU5 preparation is superior than that of Pegloticase.

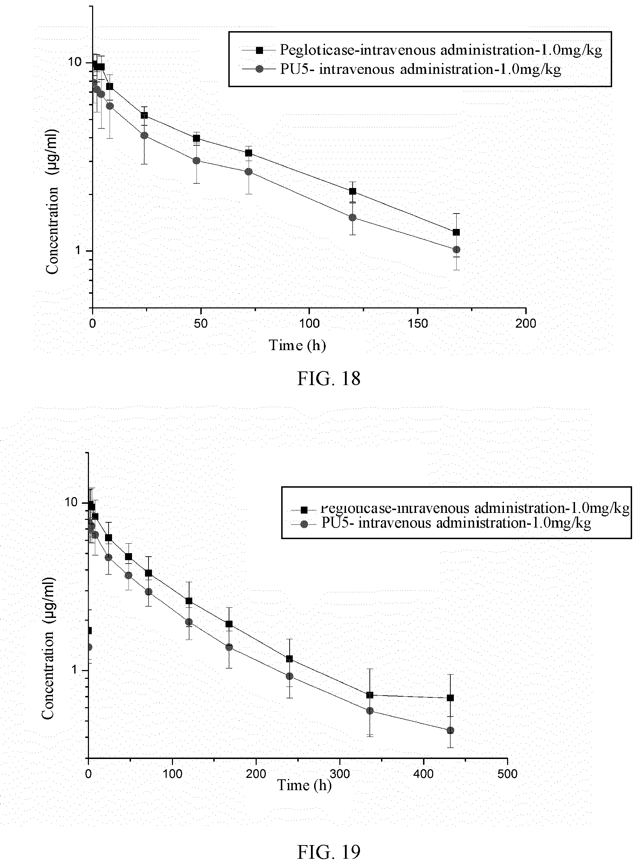

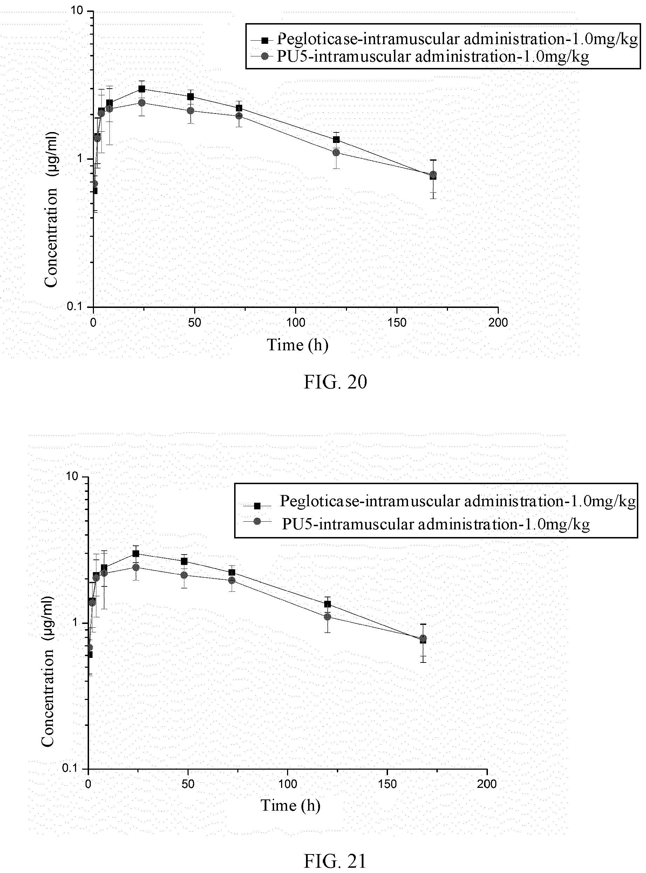

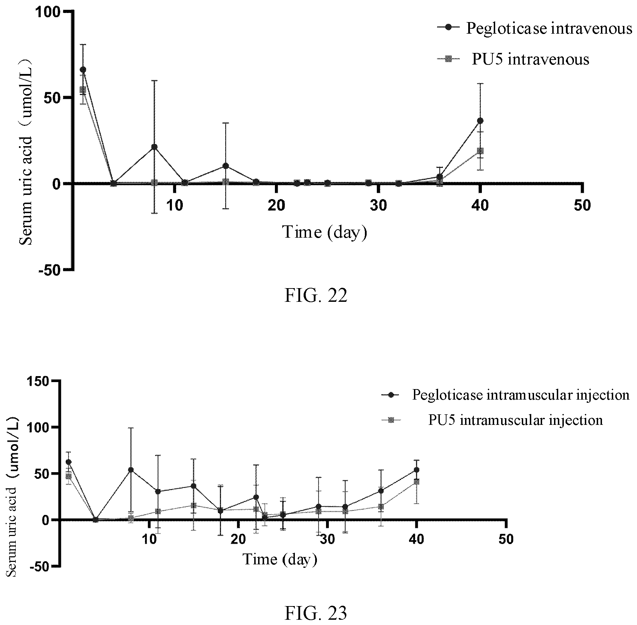

The results are shown in Tables 17 to 19 and FIGS. 18 to 22.

Table 17 Results of average pharmacokinetic parameters after continuous intravenous injection of Pegloticase and a pegylated uricase injection in SD rats

| Experiment time | Dose | Gender | Parameter | $t_{1/2}$ | $T_{max}$ | $C_{max}$ | $AUC_{last}$ | $AUC_{0-\infty}$ | Vz | Cl | $MRT_{last}$ |
|---|---|---|---|---|---|---|---|---|---|---|---|
| | Drug name | | | (h) | (h) | ($\mu$g/mL) | (h*$\mu$g/mL) | (h*$\mu$g/mL) | (mL/kg) | (mL/h/kg) | (h) |
| Day 1 | 1.0 mg/kg Pegloticase | Male | N | 4 | 4 | 4 | 4 | 4 | 4 | 4 | 4 |
| | | | Mean | 72.17 | 2.13 | 11.12 | 632.58 | 778.65 | 133.74 | 1.29 | 56.91 |
| | | | SD | 4.53 | 1.44 | 0.28 | 25.32 | 45.00 | 4.49 | 0.07 | 1.17 |
| | | Female | N | 4 | 4 | 4 | 4 | 4 | 4 | 4 | 4 |
| | | | Mean | 66.24 | 0.50 | 8.84 | 514.21 | 633.80 | 149.07 | 1.59 | 54.93 |
| | | | SD | 17.91 | 0.00 | 1.07 | 50.98 | 64.84 | 27.23 | 0.16 | 7.58 |
| | | Female + male | N | 8 | 8 | 8 | 8 | 8 | 8 | 8 | 8 |
| | | | Mean | 69.21 | 1.31 | 9.98 | 573.40 | 706.22 | 141.41 | 1.44 | 55.92 |
| | | | SD | 12.51 | 1.28 | 1.42 | 73.43 | 93.08 | 19.84 | 0.20 | 5.13 |
| | 1.0 mg/kg PU5 preparation | Male | N | 4 | 4 | 4 | 4 | 4 | 4 | 4 | 4 |
| | | | Mean | 67.98 | 1.75 | 9.47 | 527.73 | 634.48 | 158.66 | 1.60 | 55.50 |
| | | | SD | 8.87 | 1.66 | 0.91 | 91.07 | 91.12 | 39.53 | 0.22 | 0.89 |
| | | Female | N | 4 | 4 | 4 | 4 | 4 | 4 | 4 | 4 |
| | | | Mean | 79.29 | 1.38 | 6.42 | 369.05 | 481.09 | 238.12 | 2.16 | 59.68 |
| | | | SD | 17.07 | 1.75 | 0.61 | 49.11 | 98.31 | 15.34 | 0.53 | 2.84 |
| | | Female + male | N | 8 | 8 | 8 | 8 | 8 | 8 | 8 | 8 |
| | | | Mean | 73.63 | 1.56 | 7.94 | 448.39 | 557.79 | 198.39 | 1.88 | 57.59 |
| | | | SD | 13.97 | 1.59 | 1.78 | 108.54 | 120.10 | 50.74 | 0.48 | 2.96 |

| Experiment time | Dose | Gender | Parameter | $t_{1/2}$ | $T_{max}$ | $C_{max}$ | $AUC_{last}$ | $AUC_{0-\infty}$ | Vz | Cl | $MRT_{last}$ |
|---|---|---|---|---|---|---|---|---|---|---|---|
| | Drug name | | | (h) | (h) | (μg/mL) | (h*μg/mL) | (h*μg/mL) | (mL/kg) | (mL/h/kg) | (h) |
| Day 22 | 1.0 mg/kg Pegloticase | Male | N | 4 | 4 | 4 | 4 | 4 | 4 | 4 | 4 |
| | | | Mean | 135.63 | 1.75 | 12.33 | 1159.18 | 1300.99 | 149.28 | 0.78 | 118.29 |
| | | | SD | 40.45 | 1.66 | 0.94 | 134.20 | 208.65 | 28.13 | 0.13 | 9.91 |
| | | Female | N | 4 | 4 | 4 | 4 | 4 | 4 | 4 | 4 |
| | | | Mean | 96.09 | 0.88 | 8.02 | 672.95 | 747.61 | 186.89 | 1.35 | 92.46 |
| | | | SD | 7.69 | 0.75 | 0.87 | 78.50 | 78.66 | 24.21 | 0.14 | 9.52 |
| | | Female + male | N | 8 | 8 | 8 | 8 | 8 | 8 | 8 | 8 |
| | | | Mean | 115.86 | 1.31 | 10.17 | 916.07 | 1024.30 | 168.09 | 1.07 | 105.37 |
| | | | SD | 34.25 | 1.28 | 2.45 | 279.12 | 329.86 | 31.53 | 0.33 | 16.48 |
| | 1.0 mg/kg PU5 preparation | Male | N | 4 | 4 | 4 | 4 | 4 | 4 | 4 | 4 |
| | | | Mean | 149.80 | 2.25 | 9.00 | 840.78 | 947.08 | 229.16 | 1.07 | 117.99 |
| | | | SD | 24.68 | 2.02 | 0.73 | 91.96 | 104.82 | 36.69 | 0.11 | 6.88 |
| | | Female | N | 4 | 4 | 4 | 4 | 4 | 4 | 4 | 4 |
| | | | Mean | 103.90 | 1.25 | 6.63 | 576.81 | 636.52 | 236.33 | 1.63 | 101.66 |
| | | | SD | 21.25 | 0.87 | 0.72 | 128.81 | 128.02 | 17.98 | 0.39 | 20.03 |
| | | Female + male | N | 8 | 8 | 8 | 8 | 8 | 8 | 8 | 8 |
| | | | Mean | 126.85 | 1.75 | 7.82 | 708.79 | 791.80 | 232.75 | 1.35 | 109.82 |
| | | | SD | 32.51 | 1.54 | 1.44 | 175.05 | 198.21 | 27.02 | 0.40 | 16.38 |

Table 18 Results of average pharmacokinetic parameters after continuous intramuscular injection of Pegloticase and a pegylated uricase injection in SD rats

| Experiment time | Dose | Gender | Parameter | $t_{1/2}$ | $T_{max}$ | $C_{max}$ | $AUC_{last}$ | $AUC_{0-\infty}$ | Vz_F | Cl_F | $MRT_{last}$ |
|---|---|---|---|---|---|---|---|---|---|---|---|
| | Drug name | | | (h) | (h) | ($\mu$g/mL) | (h*$\mu$g/mL) | (h*$\mu$g/mL) | (mL/kg) | (mL/h/kg) | (h) |
| Day 1 | 1.0 mg/kg Pegloticase | Male | N | 4 | 4 | 4 | 4 | 4 | 4 | 4 | 4 |
| | | | Mean | 59.68 | 24.00 | 3.16 | 318.22 | 395.66 | 217.53 | 2.54 | 62.05 |
| | | | SD | 13.70 | 0.00 | 0.38 | 29.92 | 29.26 | 49.49 | 0.19 | 6.59 |
| | | Female | N | 4 | 4 | 4 | 4 | 4 | 4 | 4 | 4 |
| | | | Mean | 80.53 | 24.00 | 2.80 | 270.69 | 415.60 | 282.07 | 2.48 | 57.80 |
| | | | SD | 16.48 | 0.00 | 0.36 | 66.91 | 84.31 | 37.74 | 0.52 | 6.21 |
| | | Female + male | N | 8 | 8 | 8 | 8 | 8 | 8 | 8 | 8 |
| | | | Mean | 70.11 | 24.00 | 2.98 | 294.46 | 405.63 | 249.80 | 2.51 | 59.92 |
| | | | SD | 17.91 | 0.00 | 0.39 | 54.29 | 59.39 | 53.39 | 0.36 | 6.35 |
| | 1.0 mg/kg PU5 preparation | Male | N | 4 | 4 | 4 | 4 | 4 | 4 | 4 | 4 |
| | | | Mean | 82.25 | 6.00 | 3.06 | 290.64 | 403.89 | 294.92 | 2.50 | 61.83 |
| | | | SD | 9.79 | 2.31 | 0.25 | 34.67 | 48.73 | 29.13 | 0.31 | 6.88 |
| | | Female | N | 4 | 4 | 4 | 4 | 4 | 4 | 4 | 4 |
| | | | Mean | 70.44 | 48.00 | 2.21 | 232.11 | 306.59 | 340.32 | 3.50 | 66.28 |
| | | | SD | 13.41 | 19.60 | 0.26 | 59.53 | 90.62 | 46.97 | 1.09 | 10.28 |
| | | Female + male | N | 8 | 8 | 8 | 8 | 8 | 8 | 8 | 8 |
| | | | Mean | 76.34 | 27.00 | 2.63 | 261.38 | 355.24 | 317.62 | 3.00 | 64.06 |
| | | | SD | 12.57 | 25.90 | 0.51 | 54.89 | 85.10 | 43.56 | 0.91 | 8.44 |

(continued)

| Experiment time | Dose | Gender | Paramete r | $t_{1/2}$ | $T_{max}$ | $C_{max}$ | $AUC_{last}$ | $AUC_{0-\infty}$ | Vz_F | Cl_F | $MRT_{last}$ |
|---|---|---|---|---|---|---|---|---|---|---|---|
| | Drug name | | | (h) | (h) | (μg/mL) | (h*μg/mL) | (h*μg/mL) | (mL/kg) | (mL/h/kg) | (h) |
| Day 22 | 1.0 mg/kg Pegloticase | Male | N | 3 | 4 | 4 | 4 | 3 | 3 | 3 | 4 |
| | | | Mean | 198.20 | 25.00 | 3.18 | 486.70 | 799.90 | 353.52 | 1.35 | 112.01 |
| | | | SD | 83.85 | 18.00 | 0.85 | 298.21 | 293.71 | 23.56 | 0.42 | 60.30 |
| | | Female | N | 4 | 4 | 4 | 4 | 4 | 4 | 4 | 4 |
| | | | Mean | 97.92 | 20.00 | 2.69 | 355.77 | 427.75 | 344.97 | 2.65 | 94.51 |
| | | | SD | 33.98 | 8.00 | 0.71 | 134.18 | 155.69 | 88.41 | 1.19 | 30.45 |
| | | Female + male | N | 7 | 8 | 8 | 8 | 7 | 7 | 7 | 8 |
| | | | Mean | 140.90 | 22.50 | 2.93 | 421.23 | 587.25 | 348.64 | 2.09 | 103.26 |
| | | | SD | 76.12 | 13.17 | 0.77 | 225.23 | 283.63 | 64.14 | 1.12 | 45.20 |
| | 1.0 mg/kg PU5 preparation | Male | N | 4 | 4 | 4 | 4 | 4 | 4 | 4 | 4 |
| | | | Mean | 140.04 | 15.00 | 2.52 | 395.82 | 478.83 | 610.95 | 9.64 | 102.76 |
| | | | SD | 90.61 | 10.52 | 1.15 | 255.45 | 300.26 | 419.13 | 16.09 | 61.50 |
| | | Female | N | 4 | 4 | 4 | 4 | 4 | 4 | 4 | 4 |
| | | | Mean | 122.51 | 30.00 | 2.41 | 349.84 | 428.61 | 416.41 | 2.82 | 103.39 |
| | | | SD | 59.33 | 12.00 | 0.50 | 178.08 | 204.30 | 72.32 | 1.36 | 44.91 |
| | | Female + male | N | 8 | 8 | 8 | 8 | 8 | 8 | 8 | 8 |
| | | | Mean | 131.27 | 22.50 | 2.46 | 372.83 | 453.72 | 513.68 | 6.23 | 103.08 |
| | | | SD | 71.52 | 13.17 | 0.82 | 205.33 | 239.26 | 297.23 | 11.18 | 49.85 |

Table 19 Statistical results of uric acid in each dose group after multiple intramuscular/intravenous injections of Pegloticase and a pegylated uricase injection in SD rats (Mean + SD)

| Gender Detection time | 1.0 mg/kg Pegloticase Intravenous injection group | | 1.0 mg/kg Pegloticase Intramuscular injection group | | 1.0 mg/kg PU5 preparation Intravenous injection group | | 1.0 mg/kg PU5 preparation Intramuscular injection group | |
|---|---|---|---|---|---|---|---|---|
| | n | $\overline{X} \pm SD$ | n | $\overline{X} \pm SD$ | n | $\overline{X} \pm SD$ | n | $\overline{X} \pm SD$ |
| Male | | | | | | | | |
| Before 1st administration | 4 | 71.250 ± 19.103 | 4 | 62.250 ± 5.315 | 4 | 58.750 ± 7.632 | 4 | 50.750 ± 6.850 |
| 3 days after 1st administration | 4 | 0.250 ± 0.500 | 4 | 0.500 ± 0.577 | 4 | 0 ± 0 | 4 | 0 ± 0 |
| Before 2nd administration | 4 | 0.500 ± 0.577 | 4 | 69.750 ± 46.133 | 4 | 0.500 ± 0.577 | 4 | 0.250 ± 0.500 |
| 3 days after 2nd administration | 4 | 1.000 ± 0 | 4 | 20.750 ± 40.178 | 4 | 0.500 ± 0.577 | 4 | 17.500 ± 33.670 |
| Before 3rd administration | 4 | 1.000 ± 0 | 4 | 26.500 ± 30.116 | 4 | 1.250 ± 0.957 | 4 | 18.000 ± 32.680 |
| 3 days after 3rd administration | 4 | 1.000 ± 0.816 | 4 | 19.000 ± 37.336 | 4 | 0.500 ± 0.577 | 4 | 20.500 ± 38.336 |
| Before 4th administration | 4 | 0 ± 0 | 4 | 15.500 ± 31.000 | 4 | 0.250 ± 0.500 | 4 | 19.250 ± 37.170 |
| 1 day after 4th administration | 4 | 0.750 ± 0.500 | 4 | 1.750 ± 1.500 | 4 | 0.750 ± 0.500 | 4 | 9.750 ± 16.840 |
| 3 days after 4th administration | 4 | 0.500 ± 0.577 | 4 | 10.500 ± 21.000 | 4 | 0 ± 0 | 4 | 12.750 ± 24.838 |
| 7 days after 4th administration | 4 | 0.250 ± 0.500 | 4 | 22.250 ± 44.500 | 4 | 0 ± 0 | 4 | 16.250 ± 31.837 |
| 10 days after 4th administration | 4 | 0 ± 0 | 4 | 20.000 ± 38.670 | 4 | 0.250 ± 0.500 | 4 | 16.000 ± 30.681 |
| 14 days after 4th administration | 4 | 1.250 ± 0.500 | 4 | 29.250 ± 28.123 | 4 | 2.000 ± 0.816 | 4 | 19.250 ± 30.015 |
| 18 days after 4th administration | 4 | 25.000 ± 10.296 | 4 | 53.500 ± 14.933 | 4 | 24.000 ± 13.614 | 4 | 50.000 ± 29.833 |
| Female | | | | | | | | |
| Before 1st administration | 4 | 61.250 ± 8.057 | 4 | 63.000 ± 15.470 | 4 | 50.250 ± 7.500 | 4 | 43.250 ± 9.743 |
| 3 days after 1st administration | 4 | 0 ± 0 | 4 | 0 ± 0 | 4 | 0.250 ± 0.500 | 4 | 0 ± 0 |
| Before 2nd administration | 4 | 42.000 ± 48.132 | 4 | 38.250 ± 44.507 | 4 | 0.500 ± 0.577 | 4 | 4.000 ± 7.348 |

(continued)

| | | Female | | | | | | |
|---|---|---|---|---|---|---|---|---|
| 3 days after 2nd administration | 4 | $0.250 \pm 0.500$ | 4 | $40.250 \pm 41.080$ | 4 | $0.250 \pm 0.500$ | 4 | $0.500 \pm 0.577$ |
| Before 3rd administration | 4 | $19.500 \pm 35.01$ | 4 | $46.750 \pm 28.547$ | 4 | $1.000 \pm 0.816$ | 4 | $13.500 \pm 25.000$ |
| 3 days after 3rd administration | 4 | $1.250 \pm 0.500$ | 4 | $0.500 \pm 0.577$ | 4 | $0.750 \pm 0.500$ | 4 | $0.750 \pm 0.500$ |
| Before 4th administration | 4 | $0.250 \pm 0.500$ | 4 | $33.750 \pm 40.285$ | 4 | $0.500 \pm 0.577$ | 4 | $3.750 \pm 6.850$ |
| 1 day after 4th administration | 4 | $0.750 \pm 0.500$ | 4 | $3.000 \pm 2.708$ | 4 | $0.500 \pm 0.577$ | 4 | $1.250 \pm 0.500$ |
| 3 days after 4th administration | 4 | $0.250 \pm 0.500$ | 4 | $0 \pm 0$ | 4 | $0 \pm 0$ | 4 | $0 \pm 0$ |
| 7 days after 4th administration | 4 | $0.250 \pm 0.500$ | 4 | $6.750 \pm 12.842$ | 4 | $1.000 \pm 0$ | 4 | $1.750 \pm 1.258$ |
| 10 days after 4th administration | 4 | $0 \pm 0$ | 4 | $8.500 \pm 16.340$ | 4 | $0.250 \pm 0.500$ | 4 | $2.000 \pm 2.828$ |
| 14 days after 4th administration | 4 | $6.750 \pm 7.089$ | 4 | $33.500 \pm 19.689$ | 4 | $1.500 \pm 0.577$ | 4 | $9.500 \pm 8.699$ |
| 18 days after 4th administration | 4 | $48.000 \pm 24.993$ | 4 | $54.750 \pm 4.031$ | 4 | $14.000 \pm 6.00.$ | 4 | $32.000 \pm 13.638$ |

In the specification, descriptions with reference to the terms "an embodiment", "some embodiments", "examples", "specific examples", or "some examples", etc., mean specific features, structures, materials or characteristics described in conjunction with the embodiment or example are included in at least one embodiment or example of the present disclosure. In this specification, the above terms are illustrative, and do not necessarily refer to the same embodiment or example. Moreover, the described specific features, structures, materials or characteristics can be combined in a suitable manner in any one or more embodiments or examples. In addition, those skilled in the art can combine the different embodiments or examples and the features of the different embodiments or examples described in this specification without contradicting each other.

While embodiments of the present disclosure have been shown and described, it will be understood that the above-described embodiments are illustrative and not restrictive, and that changes, modifications, substitutions, and variations may be made by those skilled in the art without departing from the scope of the present disclosure.

**Claims**

1.   A urate oxidase preparation, comprising:

  an active ingredient selected from a polyethylene glycol-modified urate oxidase; and
  an excipient selected from a buffer reagent, wherein the buffer reagent comprises at least one of phosphate or hydrochloride.

2.   The urate oxidase preparation according to claim 1, wherein at least 11 of the following amino acid sites in the urate oxidase have a PEG modification: $T^1$, $K^3$, $K^4$, $K^{30}$, $K^{35}$, $K^{76}$, $K^{79}$, $K^{97}$, $K^{112}$, $K^{116}$, $K^{120}$, $K^{152}$, $K^{179}$, $K^{222}$, $K^{231}$, $K^{266}$, $K^{272}$, $K^{285}$, $K^{291}$, and $K^{293}$, and the amino acid sites are positioned based on an amino acid sequence set forth as SEQ ID NO: 1.

3. The urate oxidase preparation according to claim 2, wherein at least one, at least two, at least three, or four of the following four amino acid sites in the urate oxidase have a PEG modification: $K^{30}$, $K^{35}$, $K^{222}$ and $K^{231}$.

4. The urate oxidase preparation according to claim 1, wherein the polyethylene glycol has a monomethoxy group or a hydroxyl group.

5. The urate oxidase preparation according to claim 1, wherein the polyethylene glycol is of a linear or branched structure.

6. The urate oxidase preparation according to claim 1, wherein the polyethylene glycol is coupled to the urate oxidase via an amide bond.

7. The urate oxidase preparation according to any one of claims 1 to 6, wherein the polyethylene glycol is a modified polyethylene glycol, and a modification group of the modified polyethylene glycol is at least one selected from the group consisting of N-hydroxysuccinimide, N-hydroxysuccinimidyl carbonate, N-hydroxysuccinimidyl acetate, N-hydroxysuccinimidyl propionate, N-hydroxysuccinimidyl butyrate, N-hydroxysuccinimidyl succinate, and bis(p-nitrophenyl) carbonate.

8. The urate oxidase preparation according to claim 7, wherein the modification group of the modified polyethylene glycol is N-hydroxysuccinimidyl propionate.

9. The urate oxidase preparation according to claim 1, wherein the urate oxidase has an amino acid sequence set forth as any one of SEQ ID NOs: 1 to 7; or

   the urate oxidase is a polypeptide having at least 70%, at least 75%, at least 80%, at least 85%, at least 90%, at least 95%, or at least 99% identity with any of SEQ ID NOs: 1 to 7; or
   the urate oxidase is a polypeptide having an amino acid sequence set forth as any one of SEQ ID NOs: 1 to 7 in which one or more amino acids are substituted, deleted and/or added.

10. The urate oxidase preparation according to claim 1, wherein the urate oxidase has an amino acid sequence set forth as any one of SEQ ID NOs: 1 to 4.

11. The urate oxidase preparation according to claim 1, wherein the buffer reagent comprises at least one selected from the group consisting of disodium hydrogen phosphate, sodium dihydrogen phosphate, and sodium chloride.

12. The urate oxidase preparation according to claim 1, wherein the urate oxidase preparation has a pH of 7 to 9.

13. The urate oxidase preparation according to claim 12, wherein the urate oxidase preparation has a pH of 7.4 to 8.2.

14. The urate oxidase preparation according to claim 9 or 10, wherein a mass ratio of the polyethylene glycol-modified urate oxidase to the buffer reagent is (5 to 6): (6 to 37).

15. The urate oxidase preparation according to claim 14, wherein the mass ratio of the polyethylene glycol-modified urate oxidase to the buffer reagent is 6:10.

16. The urate oxidase preparation according to claim 9 or 10, wherein a mass ratio of the polyethylene glycol-modified urate oxidase to the phosphate is (5 to 6): (1 to 7), wherein the phosphate is disodium hydrogen phosphate, sodium dihydrogen phosphate.

17. The urate oxidase preparation according to claim 9 or 10, wherein a mass ratio of the polyethylene glycol-modified urate oxidase to the sodium chloride is (5 to 6): (5 to 30).

18. The urate oxidase preparation according to claim 9 or 10, wherein a mass ratio of the phosphate to the sodium chloride is (1 to 7): (5 to 30), wherein the phosphate is disodium hydrogen phosphate and/or sodium dihydrogen phosphate.

19. The urate oxidase preparation according to claim 1, wherein a dosage form of the urate oxidase preparation comprises at least one of liquid, semi-solid, or solid.

**20.** The urate oxidase preparation according to claim 1, wherein the preparation is in a single-dose form, each dose of the preparation containing 6 mg of the urate oxidase.

**21.** Use of the urate oxidase preparation according to any one of claims 1 to 20 in the manufacture of a medicament for the treatment or prevention of hyperuricemia and hyperuricemia-related diseases.

**22.** A pharmaceutical composition, comprising the urate oxidase preparation according to any one of claims 1 to 20.

**23.** The pharmaceutical composition according to claim 22, further comprising an additional drug for the treatment or prevention of hyperuricemia and hyperuricemia-related diseases.

**24.** A method for treating or preventing hyperuricemia and hyperuricemia-related diseases, comprising: administering to a subject a pharmaceutically acceptable amount of the urate oxidase preparation according to any one of claims 1 to 20 or the composition according to any one of claims 22 and 23.

FIG. 1

FIG. 2

FIG. 3

FIG. 4

FIG. 5

FIG. 6

FIG. 7

VWD1 A, Wavelength=280 nm (E:\PU5Injection\High molecular weight \201709\20170930000005.D)

Area percentage report

Sorting                          :        Signal
Product factor                   :        1.0000
Dilution factor                  :        1.0000
Sample weight                    :        400.00000    (ng/ul)     (not used in calibration)
Product factor and dilution factor are not used in the internal standard

Signal 1:VWD1   A, Wavelength=280 nm

| Peak # | Retention time [min] | Type | Peak width [min] | Peak area [mAU.s] | Peak height [mAU] | Peak area % |
|---|---|---|---|---|---|---|
| 1 | 15.277 | BV | 0.8965 | 15.35510 | 2.66366e-1 | 0.2032 |
| 2 | 17.346 | VBA | 0.7445 | 7540.78369 | 159.24133 | 99.7968 |

Total :                                    7556.13879    159.50770

FIG. 8

VWD1  A,Wavelength=280  nm (E:\PU5Injection-171030. PU5Stability test-1M 2017-10-30 14-10.28\201710300000019.D)

---

Area percentage report

---

Sorting                          :        Signal
Product factor                            :        1. 0000
Dilution factor                           :        1. 0000
Product factor and dilution factor are not used in the internal standard

Signal 1: VWD1  A, Wavelength=280  nm

| Peak # | Retention time [min] | Type | Peak width [min] | Peak area [mAU.s] | Peak height [mAU] | Peak area % |
|---|---|---|---|---|---|---|
| 1 | 14.714 | BV | 0.6759 | 3 .55794 | 8.43896e-2 | 0.0493 |
| 2 | 16.570 | VV | 0.7027 | 6138.84082 | 133.94960 | 85.0085 |
| 3 | 19.125 | VBA | 1.1450 | 1079.04541 | 13.78913 | 14.9422 |

Total  :                        7221.44417      147.82312

FIG. 9

VWD1   A,Wavelength=280  nm (E:\PU5Injection-171030. PU5Stability test-1M 2017-10-30 14-10.28\201710300000019.D)

```
--------------------------------------------------------------------
                        Area percentage report
--------------------------------------------------------------------
```

Sorting                    :      Signal
Product factor                    :        1. 0000
Dilution factor                   :        1. 0000
Product factor and dilution factor are not used in the internal standard


Signal 1: VWD1   A,Wavelength=280   nm

| Peak # | Retentiontime [min] | Type | Peak width [min] | Peak area [mAU.s] | Peak height [mAU] | Peak area % |
|---|---|---|---|---|---|---|
| 1 | 14.626 | BB | 0.5765 | 2.36490 | 6.26375e-2 | 0.0317 |
| 2 | 16.578 | BV | 0.6936 | 6645.66943 | 147.49924 | 89.2010 |
| 3 | 19.229 | VBA | 1.3338 | 802.18494 | 8.61966 | 10.7673 |

Total  :                              7450.21927      156.18154

FIG. 10

VWD1 A.Wavelength=280 nm (E:\PU5injection-171030. PU5Stability test-1M 2017-10-30 14-10.28\201710300000021.D)

---

Area percentage report

---

| Sorting | : | Signal |
|---|---|---|
| Product factor | : | 1.0000 |
| Dilution factor | : | 1.0000 |

Product factor and dilution factor are not used in the internal standard

Signal 1: VWD1 A,Wavelength=280 nm

| Peak # | Retention time [min] | Type | Peak width [min] | Peak area [mAU.s] | Peak height [mAU] | Peak area % |
|---|---|---|---|---|---|---|
| 1 | 14.612 | MF R | 0.6085 | 2.30933 | 6.32571e-2 | 0.0315 |
| 2 | 16.532 | FX R | 0.7600 | 4 58.53564 | 104.35711 | 64.8376 |
| 3 | 17.855 | VV B | 0.3472 | 146.77948 | 6.22189 | 1.9999 |
| 4 | 19.162 | VBAR | 1.0055 | 2431.53613 | 35.93070 | 33.1310 |

| Total : | | | | 7339.16059 | 146.57296 | |

FIG. 11

G1: Blank control group (n=10)
G2: Model control group (n=10)
G3: PU5 low-dose group (n=10)
G4: PU5 medium-dose group (n=10)
G5: PU5 high-dose group (n=10)

FIG. 12

FIG. 13

FIG. 14

FIG. 15

FIG. 16

FIG. 17

FIG. 18

FIG. 19

FIG. 20

FIG. 21

FIG. 22

FIG. 23

<div align="center">

**INTERNATIONAL SEARCH REPORT**

</div>

| International application No. |
|---|
| **PCT/CN2021/129071** |

**A. CLASSIFICATION OF SUBJECT MATTER**

A61K 38/44(2006.01)i; A61K 47/60(2017.01)i; A61P 19/06(2006.01)i; C12N 9/06(2006.01)i

According to International Patent Classification (IPC) or to both national classification and IPC

**B. FIELDS SEARCHED**

Minimum documentation searched (classification system followed by classification symbols)

A61K; A61P; C12N

Documentation searched other than minimum documentation to the extent that such documents are included in the fields searched

Electronic data base consulted during the international search (name of data base and, where practicable, search terms used)

数据库: WPABSC, CNTXT, OETXT, ISI WEB OF KNOWLEDGE, PUBMED, CNKI, Genbank, EMBL, China PAtents Biological Sequence Search System; 检索词: 聚乙二醇, 尿酸氧化酶, 尿酸酶, 甲氧基, 酰胺键, 琥珀酰亚胺, T1, K3, K4, K30, K35, K76, K79, K97, K112, K116, K120, K152, K179, K222, K231, K266, K272, K285, K291, K293, Polyethlene glycol, PEG, Urate Oxidase, UOX, Methoxy, Amide bond, Succinimide, SEQ ID NOs.1-7 sequence alignment.

**C. DOCUMENTS CONSIDERED TO BE RELEVANT**

| Category* | Citation of document, with indication, where appropriate, of the relevant passages | Relevant to claim No. |
|---|---|---|
| X | CN 101928704 A (HANGZHOU BIODOOR BIOTECHNOLOGY CO., LTD.) 29 December 2010 (2010-12-29)<br> see claims 1-18, embodiments 2-3, 8, figure 1 | 1, 4-8, 11-23 |
| Y | CN 101928704 A (HANGZHOU BIODOOR BIOTECHNOLOGY CO., LTD.) 29 December 2010 (2010-12-29)<br> see claims 1-18, embodiments 2-3, 8, figure 1 | 9-10 |
| A | CN 102634492 A (CHONGQING FUJIN BIO-PHARM CO., LTD.) 15 August 2012 (2012-08-15)<br> see entire document | 1-23 |
| A | CN 105087530 A (YANGZHOU AIDEA BIOTECH CO., LTD.) 25 November 2015 (2015-11-25)<br> see entire document | 1-23 |
| A | CN 101302501 A (LIU, Guoan; YANG, Zhong) 12 November 2008 (2008-11-12)<br> see entire document | 1-23 |

☑ Further documents are listed in the continuation of Box C.          ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **26 January 2022** | **10 February 2022** |

| Name and mailing address of the ISA/CN | Authorized officer |
|---|---|
| **China National Intellectual Property Administration (ISA/ CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

| International application No. |
| --- |
| **PCT/CN2021/129071** |

**C.    DOCUMENTS CONSIDERED TO BE RELEVANT**

| Category* | Citation of document, with indication, where appropriate, of the relevant passages | Relevant to claim No. |
| --- | --- | --- |
| Y | "uricase precursor [Sus scrofa]"<br>*NCBI Reference Sequence: NP_999435.1,* 11 October 2020 (2020-10-11),<br>see amino acid sequence and related information | 9-10 |

**INTERNATIONAL SEARCH REPORT**

| International application No. |
| --- |
| **PCT/CN2021/129071** |

Box No. I          Nucleotide and/or amino acid sequence(s) (Continuation of item 1.c of the first sheet)

1.  With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2.  ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet) (January 2015)

**INTERNATIONAL SEARCH REPORT**

| International application No. |
| --- |
| **PCT/CN2021/129071** |

**Box No. II    Observations where certain claims were found unsearchable (Continuation of item 2 of first sheet)**

This international search report has not been established in respect of certain claims under Article 17(2)(a) for the following reasons:

1. ☑ Claims Nos.: **24**
because they relate to subject matter not required to be searched by this Authority, namely:

    [1]   A method for treatment of the human or animal body.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

INTERNATIONAL SEARCH REPORT
Information on patent family members

International application No.

**PCT/CN2021/129071**

| Patent document cited in search report | | | Publication date (day/month/year) | Patent family member(s) | | | Publication date (day/month/year) |
|---|---|---|---|---|---|---|---|
| CN | 101928704 | A | 29 December 2010 | None | | | |
| CN | 102634492 | A | 15 August 2012 | WO | 2012109975 | A1 | 23 August 2012 |
| | | | | EP | 2684950 | A1 | 15 January 2014 |
| | | | | EP | 2684950 | A4 | 07 January 2015 |
| | | | | US | 2014065123 | A1 | 06 March 2014 |
| | | | | US | 9193967 | B2 | 24 November 2015 |
| | | | | CN | 102634492 | B | 10 June 2015 |
| CN | 105087530 | A | 25 November 2015 | None | | | |
| CN | 101302501 | A | 12 November 2008 | CN | 101302501 | B | 04 July 2012 |

Form PCT/ISA/210 (patent family annex) (January 2015)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7056713 B1, Michael H, Susan J. K.

- CN 1264575 C, Savient

**Non-patent literature cited in the description**

- **SUZUKI K ; SAKASEGAWA S ; MISAKI H ; SUGIYAMA M.** *J Biosci Bioeng,* 2004, vol. 98, 153-158
- **RETAILLEAU P ; COLLOC'H ; DENIS V ; FRANCOISE B.** *Acta Cryst,* 453-462
- **BAYOL A et al.** *Biophys Chem,* 1995, vol. 54, 229-235

- **BAYOL A et al.** *Biophys Chem.,* 1995, vol. 54, 229-235
- **HUANGS H ; WU T K.** *Eur J Biochem.,* 2004, vol. 271, 517-523
- **LEE C C ; WU X ; GIBBS R A ; COOK R G ; MUZNY D M ; CASKEYC T.** *Science,* 1988, vol. 239, 1288-1291